(19) Europäisches Patentamt
European Patent Office
Office européen des brevets

(11) EP 3 134 446 B1

(12) EUROPÄISCHE PATENTSCHRIFT

(45) Veröffentlichungstag und Bekanntmachung des Hinweises auf die Patenterteilung:
**24.04.2019 Patentblatt 2019/17**

(21) Anmeldenummer: **15711757.3**

(22) Anmeldetag: **24.03.2015**

(51) Int Cl.:
***C08F 290/06*** (2006.01) ***G03F 7/00*** (2006.01)
***G03F 7/24*** (2006.01) ***G11B 7/245*** (2006.01)
***G11B 7/24044*** (2013.01)

(86) Internationale Anmeldenummer:
**PCT/EP2015/056178**

(87) Internationale Veröffentlichungsnummer:
**WO 2015/161969 (29.10.2015 Gazette 2015/43)**

(54) **AROMATISCHE GLYKOLETHER ALS SCHREIBMONOMERE IN HOLOGRAPHISCHEN PHOTOPOLYMER-FORMULIERUNGEN**

AROMATIC GLYCOL ETHERS AS CO-MONOMERS IN HOLOGRAPHIC PHOTOPOLYMER FORMULAS

ÉTHER DE GLYCOL AROMATIQUE COMME MONOMÈRE D’ENREGISTREMENT DANS DES FORMULES PHOTO-POLYMÈRES HOLOGRAPHIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.04.2014 EP 14165956**

(43) Veröffentlichungstag der Anmeldung:
**01.03.2017 Patentblatt 2017/09**

(73) Patentinhaber: **Covestro Deutschland AG**
**51373 Leverkusen (DE)**

(72) Erfinder:
- **FÄCKE, Thomas**
  **51375 Leverkusen (DE)**
- **BRUDER, Friedrich-Karl**
  **47802 Krefeld (DE)**
- **RÖLLE, Thomas**
  **51381 Leverkusen (DE)**
- **WEISER, Marc-Stephan**
  **51377 Leverkusen (DE)**
- **HÖNEL, Dennis**
  **53909 Zülpich-Wichterich (DE)**
- **BERNETH, Horst**
  **51373 Leverkusen (DE)**
- **WALZE, Günther**
  **51377 Leverkusen (DE)**
- **HAGEN, Rainer**
  **51377 Leverkusen (DE)**

(74) Vertreter: **Levpat**
**c/o Covestro AG**
**Gebäude 4825**
**51365 Leverkusen (DE)**

(56) Entgegenhaltungen:
**EP-A1- 1 674 940** **EP-A1- 2 401 998**
**WO-A1-2011/095442**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft eine Photopolymer-Formulierung umfassend Schreibmonomere, Matrixpolymere und einen Photoinitiator. Weiterhin sind ein unbelichtetes holographisches Medium, erhältlich unter Verwendung einer erfindungsgemäßen Photopolymer-Formulierung, und ein belichtetes holographisches Medium, erhältlich indem ein Hologramm in ein erfindungsgemäßes unbelichtetes holographisches Medium einbelichtet wird, Gegenstände der Erfindung. Ebenfalls Gegenstände der Erfindung sind eine optische Anzeige umfassend ein erfindungsgemäßes belichtetes holographisches Medium, die Verwendung eines erfindungsgemäßen belichteten holographischen Mediums zur Herstellung von Chipkarten, Ausweisdokumenten, 3D-Bildern, Produktschutzetiketten, Labeln, Banknoten oder holografisch optischen Elementen sowie spezielle Aromatische Glycolether.

Für die Verwendungen von Photopolymer-Formulierungen spielt der durch die holographische Belichtung im Photopolymer erzeugte Brechungsindexkontrast $\Delta n$ die entscheidende Rolle. Bei der holographischen Belichtung wird das Interferenzfeld aus Signal- und Referenzlichtstrahl (im einfachsten Fall das zweier ebener Wellen) durch die lokale Photopolymerisation von z.B. hochbrechenden Acrylate an Orten hoher Intensität im Interferenzfeld in ein Brechungsindexgitter abgebildet. Das Brechungsindexgitter im Photopolymeren (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz, im folgenden DE wie Diffraction Efficiency, genannt. Im einfachsten Fall eines Hologramms, das aus der Überlagerung zweier ebener Wellen entsteht, ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die Lichtmenge des Referenzlichtes, die notwendig ist, um das Signal mit einer festen Helligkeit sichtbar zu machen.

Hochbrechende Acrylate sind in der Lage, Brechungsindexgitter mit hoher Amplitude zwischen Bereichen mit niedrigem Brechungsindex und Bereichen mit hohem Brechungsindex zu erzeugen und damit in Photopolymer-Formulierungen Hologramme mit hohem DE und hohem $\Delta n$ zu ermöglichen. Dabei ist zu beachten, dass DE vom Produkt aus $\Delta n$ und der Photopolymerschichtdicke d abhängt. Die Breite des Winkelbereiches bei dem das Hologramm z.B. bei monochromatischer Beleuchtung sichtbar (rekonstruiert) wird, hängt nur von der Schichtdicke d ab.

Bei Beleuchtung des Hologramms mit z.B. weißem Licht hängt die Breite des spektralen Bereiches, der zur Rekonstruktion des Hologramms beitragen kann, ebenfalls nur von der Schichtdicke d ab. Dabei gilt je kleiner d desto größer die jeweiligen Akzeptanzbreiten. Will man daher helle und leicht sichtbare Hologramme herstellen, ist ein hohes $\Delta n$ und eine geringe Dicke d anzustreben und zwar so, dass DE möglichst groß wird. Das heißt je höher $\Delta n$ wird, desto mehr Freiraum zur Gestaltung der Schichtdicke d für helle Hologramme erreicht man ohne Verlust an DE. Daher kommt der Optimierung von $\Delta n$ bei der Optimierung von Photopolymer-Formulierungen eine herausragenden Bedeutung zu (P. Hariharan, Optical Holography, 2nd Edition, Cambridge University Press, 1996).

Aus der WO 2008/125229 sind Photopolymer-Formulierungen bekannt, die hochmolekulare, mono- und difunktionelle Schreibmonomere enthalten. In Medien aus diesen Formulierungen können Reflexions-Hologramme geschrieben werden, die sich beispielsweise gut für die Datenspeicherung eignen. Allerdings treten bei der Herstellung und Verarbeitung der Formulierungen Probleme auf: So weisen die enthaltenen Schreibmonomere eine große Viskosität bzw. hohe $T_G$-Werte ($T_G$ = Glasübergangstemperatur) auf. Dies bedingt, dass eine gleichmäßige Verteilung der Schreibmonomere in der Photopolymer-Formulierung und einem daraus hergestellten Medium schwierig zu realisieren ist. Außerdem kann es bei der Verwendung der bekannten Formulierungen in der Polymermatrix zur Bildung von Schreibmonomer-Agglomeraten kommen, was die Qualität der Medien bzw. der darin eingeschriebenen Hologramme erheblich beeinträchtigt. In derartigen Fällen werden die holographischen Materialien trüb.

Eine besondere Form von Hologrammen sind Transmissions-Hologramme, die sich dadurch auszeichnen, dass beim Erzeugen der Hologramme der Referenz- und der Objektstrahl das holografische Medium von derselben Seite bestrahlen. Transmissions-Hologramme finden vielfältige Anwendungen. Insbesondere ist hier die Lichtlenkung als diffraktives optisches Element zu nennen. Derartige optische Element können in anspruchsvollen Anwendungen wie der Spektroskopie oder der Astronomie zum Einsatz kommen. Ebenso eignen sie sich zur Verwendung in elektronischen Displays, wie z.B. in 3D-Displays.

Bedingt durch die Geometrie der zur Interferenz gebrachten Objekt- und Signalstrahlen ist der Gitterabstand in Transmissions-Hologrammen im Vergleich zu Reflexions-Hologrammen groß. Je nach Wellenlänge kann er zwischen 500-1000 nm betragen. Da der Mechanismus der Hologrammbildung in dem Photopolymer auf der Diffusion der Schreibmonomere beruht, sind Schreibmonomere notwendig, die bei dem für Transmissions-Hologramme üblichen großen Gitterabstand hinreichend weit diffundieren können. Dies ist jedoch Voraussetzung, um einen hoher Brechungsindexkontrast ($\Delta n$) ermöglichen zu können. Die aus dem Bereich der Reflexionshologramme bekannten Photopolymere sind hierfür häufig nicht geeignet, bzw. führen nicht zu einem ausreichenden hohen Brechungsindexkontrast.

In der WO 2012/020061 werden spezielle (Meth)acrylatgruppen haltige Schreibmonomere auf Basis von Thioethern sowie diese enthaltende Photopolymer-Formulierungen und Medien insbesondere zur Aufnahme von Transmissionshologrammen beschrieben. Allerdings weist das zur Herstellung der Schreibmonomere verwendete Verfahren Nachteile auf: So werden in einem ersten Schritt Epoxide und Thiole unter Katalyse zu hydroxy-funktionellen Thioethern umgesetzt, die dann in einem zweiten Schritt mit isocyanatfunktionellen Acrylaten zur Reaktion gebracht werden. Die Reaktion der Thiole ist jedoch eine vergleichsweise langsame Reaktion und ein vollständiger Umsatz der meist stark geruchsintensiven Edukte ist schwierig zu realisieren. Auch die Handhabung dieser Materialien und die Reinigung von Reaktionsgefäßen im Produktionsumfeld bringen einen höheren Aufwand mit sich. Weiterhin inhibieren nicht umgesetzte freie Thiole die radikalische Polymerisation, so daß es wünschenswert wäre, Alternativen zu den beschriebenen Produkten zu identifizieren, die thiolfrei hergestellt werden können.

Weiterhin ist bei den Schreibmonomeren der WO 2012/020061 und der WO 2008/125199 nachteilig, dass diese hochviskos sind bzw. pastöse Massen darstellen. Hierdurch wird die Herstellung von Photopolymer-Formulierungen erschwert, da eine effiziente, schnelle homogene Vermischung der Komponenten zu erreichen ist. Zudem sind diese hochviskosen Schreibmonomere schlecht zu filtrieren und generell unvorteilhaft zu handhaben. Weiterhin kann es auch nötig seindann organische Lösemittel einzusetzen, was aus Arbeitsschutz- und Umweltschutzgründen nachteilig ist. Darüber hinaus ist die Verwendung von Lösemitteln bei Herstellung von holografischen Medien insbesondere mit hohen Schichtdicken nachteilig, da sie nur mit großerem Aufwand beispielsweise aus Medien in Form von Filmen entfernt werden können, wobei es dann aber wiederum zu einer Beeinträchtigung der Qualität, etwa in Form von Störungen der Oberfläche, kommen kann. Solche Störungen sind jedoch in vielen Fällen nicht hinnehmbar, da die Medien dann nicht mehr die ihnen zugedachte hoch präzisen optischen Funktionen realisieren können.

EP 2401998 A1 offenbart dentale Zusammensetzungen, ein Kit-of-parts und die Verwendung der dentalen Zusammensetzungen.

WO 2011/095442 A2 offenbart eine Photopolymer-Formulierung mit Ester-basierten Schreibmonomeren.

EP 1674940 A1 offenbart einen elektrophotografischen Photorezeptor, ein bildgebendes Verfahren, eine Apparatur zur Bildgebung und eine Kartusche hierfür zum Nutzen des elektrophotografischen Photorezeptors.

Weiterhin ist generell bei der Entwicklung von Photopolymer-Formulierungenn von Vorteil, wenn die Schreibmonomere in den weiteren Komponenten eine hohe Löslichkeit aufweisen. So können in diesem Fall die Mengenverhältnisse der Komponenten in der Formulierung in einem größeren Bereich variiert werden, was die Anpassung an spezifische Anwendungen erheblich erleichtert bzw. überhaupt erst ermöglicht.

Aufgabe der vorliegenden Erfindung war es daher, eine Photopolymer-Formulierung der eingangs genannten Art bereit zu stellen, in der sich das enthaltene Schreibmonomer schnell und einfach ohne Zusatz von Lösemitteln löst. Darüber hinaus sollte sich das enthaltene Schreibmonomer ohne Verwendung von Thiolen herstellen lassen. Schließlich sollten aus der Photopolymer-Formulierung holographische Medien erhalten werden können, die trübungsarm sind, d.h. bei einer Photopolymerschichtdicke > 6 μm einem Haze von weniger als 5% aufweisen, und in die Transmissionshologramme mit hohem Brechungsindexkontrast (Δn) von mehr als 0,02 einbelichtet werden können.

Diese Aufgabe wird durch eine Photopolymer-Formulierung umfassend

A) als Schreibmonomer mindestens einen aromatischer Glycolether der allgemeinen Formel (I)

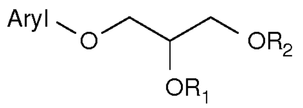

(I)

in der Aryl ein substituierter oder unsubstituierter aromatischer Rest ist, und $R_1$ und $R_2$ jeweils unabhängig voneinander ein Rest der Formel (II) oder (III)

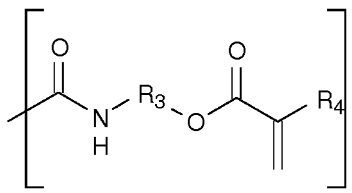

(II)

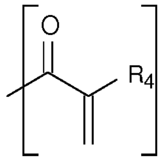
(III)

sind, in denen

$R_3$ ein organischer Rest mit bis zu 6 Kohlenstoffatomen ist, der Sauerstoff- und / oder Schwefelatome enthalten kann, und
$R_4$ ein Rest ausgewählt aus der Gruppe -H, $-CH_3$ ist;

B) Matrixpolymere;

C) einen Photoinititiator,

gelöst.

Es wurde überraschenderweise gefunden, dass mit Hilfe der erfindungsgemäßen Photopolymer-Formulierungen holographische Medien erhältlich sind, die sich insbesondere zur Aufnahme von Transmissions-Hologrammen eignen. Diese Medien weisen einen niedrigen Haze auf und liefern darüber hinaus auch einen hohen Brechungsindexkontrast ($\Delta$n). Weiterhin lassen sich die enthaltenen Schreibmonomere ohne Verwendung von Thiolen herstellen und können schnell und ohne Zusatz von Lösemitteln in der Formulierung gelöst werden.

Die Verbindungen der Formel (I) können beispielsweise in einem zwei stufigen Verfahren hergestellt werden. Dabei kann zunächst in einem ersten Schritt ein ein- bis dreifach funktioneller Arylglycidether mit (Meth)acrylsäure umgesetzt werden. Die Addition bei Anwesenheit von geeigneten Katalysatoren wie z.B. Phosphinen, Phosphoniumverbindungen, Broenstedtsäuren oder Aminen erfolgt bevorzugt an der sterisch weniger gehinderten Seite des Oxirans, so dass mehr sekundäre als primäre Alkohole gebildet werden. Anschließend können die entstandenen Alkohole an Isocyanatoalkyl(meth)acrylat addiert werden, wobeiüberwiegend Verbindungen mit $R_1$= (Meth)acrylsäure und $R_2$= Carbonylaminoalkyl(meth)acrylat erhalten werden. Im nachfolgenden Formelschema ist die Synthesestrategie noch einmal gezeigt.

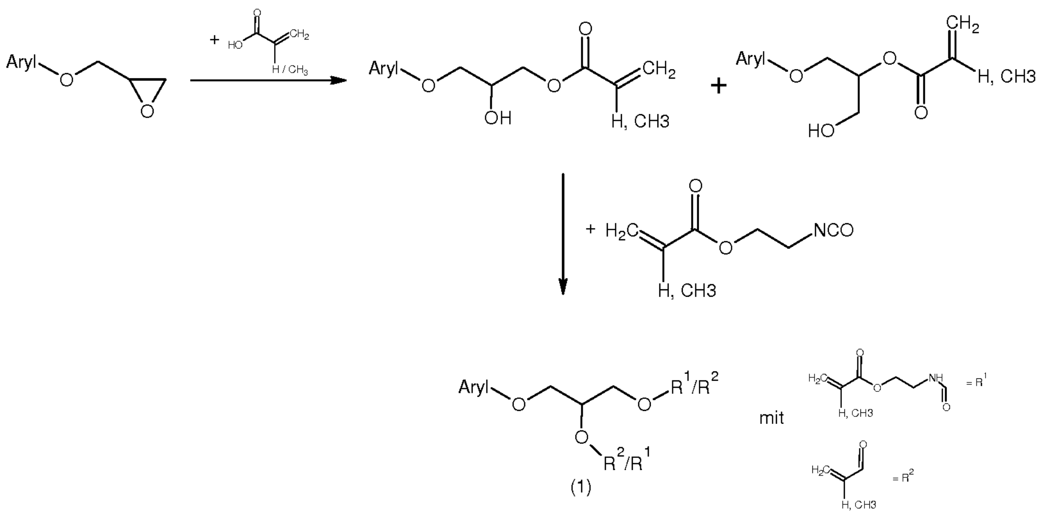

Es ist ebenfalls möglich, spezielle Katalysatoren einzusetzen, die die Regioselektivität der Addition der (Meth)acrylsäure an das Epoxid beeinflussen. So können unter Verwendung von Cobalt (III) Komplexen als Katalysator ausschließlich sekundäre Alkohole hergestellt werden (siehe A Bukowska et al., Journal of Molecular Catalysis A: Chemical 225 (2005) 7-10 und die darin zitierte Literatur). Es können also auch regioisomerenreine Produkte erhalten werden.

Gemäß einer vorteilhaften Ausführungsform ist vorgesehen, dass in der Verbindung der Formel (I) der Rest Aryl 5 bis 21, bevorzugt 5 bis 18, weiter bevorzugt 6 bis 16 und besonders bevorzugt 6 bis 12 und ganz besonders bevorzugt 6 bis 10 Kohlenstoff- und / oder Heteroatome im aromatischen System umfasst.

Ebenfalls vorteilhaft ist, wenn in der Verbindung der Formel (I) der Rest Aryl mit 1 bis 5 und bevorzugt 1 bis 3 gleichen oder unterschiedlichen Substituenten ausgewählt aus der Gruppe n-Alkyl, verzweigtes Alkyl, Alkyloxy, Phenyl, Benzyl, Phenylalkyl, Naphtyl, Methylthiyl, Ethylthiyl, Alkylthiyl, Alkylthioalkyl, Phenoxy, Phenylthiyl, Napthylthiyl, Fluor, Chlor, Brom und/oder Iod und bevorzugt mit 1 bis 3 gleichen oder unterschiedlichen Substituenten ausgewählt aus der Gruppe Methyl, Ethyl, Thiomethyl, Methoxy, Phenyl substituiert ist.

Ganz besonders bevorzugt ist, wenn in der Verbindung der Formel (I) der Rest Aryl ausgewählt aus der Gruppe Phenyl, Methylphenyl, Ethylphenyl, Thiomethylphenyl, Methoxyphenyl, Biphenyl und Naphtyl ist.

Vorteilhaft ist auch, wenn $R_3$ ein Rest ausgewählt aus der Gruppe $-CH_2-$, $-CH_2CH_2-$,$-CH(CH_3)CH_2-$, $-CH_2CH(CH_3)-$, $-CH_2CH_2CH_2-$, $-CH_2CH_2CH_2CH_2-$, $-CH_2CHOCH_2CH_2-$,$-CH_2CH_2OCH_2CH_2OCH_2CH_2-$ und bevorzugt ein Rest ausgewählt aus der Gruppe $-CH_2CH_2-$, $-CH(CH_3)CH_2-$, $-CH_2CH(CH_3)-$, $-CH_2CH_2CH_2CH_2-$, und ganz besonders bevorzugt-$CH_2CH_2-$ ist.

Gemäß einer weiteren Ausführungsform ist vorgesehen, dass der Rest $R_1$ ein Rest der Formel (II) und der Rest $R_2$ ein Rest der Formel (III) ist, wobei der Rest $R_3$ insbesondere ein-$CH_2CH_2-$ Rest sein kann. Alternativ kann der Rest $R_1$ ein Rest der Formel (III) und der Rest $R_2$ ein Rest der Formel (II) sein, wobei der Rest $R_3$ insbesondere ein $-CH_2CH_2-$ Rest sein kann.

Als Matrixpolymere B) können amorphe Thermoplaste wie z.B. Polyacrylate, Polymethylmethacrylate oder Copolymere von Methylmethacrylat, Methacrylsäure oder andere Alkylacrylate und Alkylmethacrylate sowie Acrylsäure, wie z.B. Polybutylacrylat, weiterhin Polyvinylacetat und Polyvinylburyrat seine partiell hydrolysierten Derivate wie Polyvinylalkohole sowie Copolymerisate mit Ethylene und/oder weiteren (Meth)acrylaten, Gelatine, Celluloseester und Celluloseether wie Methylcellulose, Celluloseacetobutyrat, Silikone, wie z.B. Polydimethylsilicon, Polyurethane, Polybutadiene und Polyisoprene, sowie Polyethylenoxide, Epoxyharze, insbesondere aliphatische Epoxyharze, Polyamide, Polycarbonate sowie die in US 4994347A und darin zitierten Systeme verwendet werden.

Besonders bevorzugt ist aber, wenn die Matrixpolymere Polyurethane sind.

Besonders bevorzugt ist, wenn die Matrixpolymere vernetzt sind. Insbesondere bevorzugt ist dabei, wenn die Matrixpolymere dreidimensional vernetzt sind. Epoxyharze können kationisch mit sich selbst vernetzt werden. Weiterhin können auch Säure/anhydride, Amine, Hydoxyalkylamide sowie Thiole als Vernetzer eingesetzt werden.

Silicone können sowohl als Einkomponentensysteme durch Kondensation bei Anwesenheit von Wasser (und ggf. unter Broenstedtsäurenkatalyse) oder als zweikomponentige Systeme durch Zugabe von Kieselsäureester oder zinnorganische Verbindungen vernetzt werden. Ebenso ist die Hydrosilylierung in Vinyl-Silansystemen möglich.

Ungesättigte Verbindungen, wie z.B. Acryloyl funktionelle Polymere oder ungesättigte Ester können mit Aminen oder Thiolen vernetzt werden. Eine kationische Vinyletherpolymerisation ist auch möglich.

Ganz besonders bevorzugt ist, wenn die Matrixpolymere vernetzte und insbesondere dreidimensional vernetzte Polyurethane sind.

Die Polyurethane sind insbesondere durch Umsetzung wenigstens einer Polyisocyanat-Komponente a) mit wenigstens einer Isocyanat-reaktiven-Komponente b) erhältlich.

Die Polyisocyanat-Komponente a) umfasst wenigstens eine organische Verbindung mit wenigstens zwei NCO-Gruppen. Bei diesen organischen Verbindungen kann es sich insbesondere um monomere Di- und Triisocyanate, Polyisocyanate und / oder NCO-funktionelle Prepolymere handeln. Die Polyisocyanat-Komponente a) kann auch Mischungen monomerer Di- und Triisocyanate, Polyisocyanate und / oder NCO-funktioneller Prepolymere enthalten oder daraus bestehen.

Als monomere Di- und Triisocyanate können alle dem Fachmann an sich gut bekannten Verbindungen oder deren Mischungen eingesetzt werden. Diese Verbindungen können aromatische, araliphatische, aliphatische oder cycloaliphatische Strukturen aufweisen. In untergeordneten Mengen können die monomeren Di- und Triisocyanate auch Monoisocyanate, d.h. organische Verbindungen mit einer NCO-Gruppe umfassen.

Beispiele für geeignete monomere Di- und Triisocyanate sind 1,4-Butandiisocyanat, 1,5-Pentandiisocyanat, 1,6-Hexandiisocyanat (Hexamethylendiisocyanat, HDI), 2,2,4-Trimethylhexamethylendiisocyanat und / oder 2,4,4-Trimethylhexamethylendiisocyanat (TMDI), Isophorondiisocyanat (IPDI), 1,8-Diisocyanato-4-(isocyanatomethyl)-octan, Bis-(4,4'-isocyanatocyclohexyl)methan und / oder Bis-(2',4-isocyanatocyclohexyl)methan und / oder deren Mischungen beliebigen Isomerengehalts, 1,4-Cyclohexandiisocyanat, die isomeren Bis-(isocyanatomethyl)cyclohexane, 2,4- und / oder 2,6-Diisocyanato-1-methylcyclohexan (Hexahydro-2,4- und / oder 2,6-toluylendiisocyanat, $H_6$-TDI), 1,4-Phenylendiisocyanat, 2,4- und / oder 2,6-Toluylendiisocyanat (TDI), 1,5-Naphthylendiisocyanat (NDI), 2,4'- und / oder 4,4'-Diphenylmethandiisocyanat (MDI), 1,3-Bis(isocyanatomethyl)benzol (XDI) und / oder das analoge 1,4-Isomere oder beliebige Mischungen der vorgenannten Verbindungen.

Geeignete Polyisocyanate sind Verbindungen mit Urethan-, Harnstoff-, Carbodiimid-, Acylharnstoff-, Amid-, Isocyanurat-, Allophanat-, Biuret-, Oxadiazintrion-, Uretdion- und/oder Iminooxadiazindionstrukturen, die aus den vorgenannten Di- oder Triisocyanaten erhältlich sind.

Besonders bevorzugt handelt es sich bei den Polyisocyanaten um oligomerisierte aliphatische und / oder cycloaliphatische Di- oder Triisocyanate, wobei insbesondere die oben stehenden aliphatischen und / oder cycloaliphatischen Di- oder Triisocyanate verwendet werden können.

Ganz besonders bevorzugt sind Polyisocyanate mit Isocyanurat-, Uretdion- und / oder Iminooxadiazindion-Strukturen sowie Biurete basierend auf HDI oder deren Mischungen.

Geeignete Prepolymere enthalten Urethan- und / oder Harnstoff-Gruppen sowie gegebenenfalls weitere durch Modifizierung von NCO-Gruppen entstandene Strukturen wie oben genannt. Derartige Prepolymere sind beispielsweise durch Umsetzung der oben genannten monomeren Di- und Triisocyanate und / oder Polyisocyanaten a1) mit isocyanatreaktiven Verbindungen b1) erhältlich.

Als isocyanatreaktive Verbindungen b1) können Alkohole, Amino oder MercaptoVerbindungen, bevorzugt Alkohole, verwendet werden. Dabei kann es sich insbesondere um Polyole handeln. Ganz besonders bevorzugt können als isocyanatreaktive Verbindung b1) Polyester-, Polyether-, Polycarbonat-, Poly(meth)acrylat- und/oder Polyurethan-Polyole verwendet werden.

Als Polyesterpolyole sind beispielsweise lineare Polyesterdiole oder verzweigte Polyesterpolyole geeignet, die in bekannter Weise durch Umsetzung von aliphatischen, cycloaliphatischen oder aromatischen Di- bzw. Polycarbonsäuren bzw. ihren Anhydriden mit mehrwertigen Alkoholen einer OH-Funktionalität $\geq 2$ erhalten werden können. Beispiele für geeignete Di- bzw. Polycarbonsäuren sind mehrwertige Carbonsäuren wie Bernstein-, Adipin-, Kork-, Sebacin-, Decandicarbon-, Phthal-, Terephthal-, Isophthal- Tetrahydrophthal- oder Trimellithsäure sowie Säureanhydride wie Phthal-, Trimellith- oder Bernsteinsäureanhydrid oder deren beliebige Gemische untereinander. Die Polyesterpolyole können auch auf natürlichen Rohstoffen wie Rizinusöl basieren. Es ist ebenfalls möglich, dass die Polyesterpolyole auf Homo- oder Mischpolymerisaten von Lactonen basieren, die bevorzugt durch Anlagerung von Lactonen bzw. Lactongemischen wie Butyrolacton, $\varepsilon$-Caprolacton und / oder Methyl-$\varepsilon$-caprolacton an hydroxyfunktionelle Verbindungen wie mehrwertige Alkohole einer OH-Funktionalität $\geq 2$ beispielsweise der nachstehend genannten Art erhalten werden können.

Beispiele für geeignete Alkohole sind alle mehrwertigen Alkohole wie z.B. die $C_2$ - $C_{12}$-Diole, die isomeren Cyclohexandiole, Glycerin oder deren beliebige Gemische untereinander.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkohole einer OH-Funktionalität $\geq 2$, bevorzugt Butandiol-1,4, Hexandiol-1,6 und / oder 3-Methylpentandiol. Auch Polyesterpolyole können zu Polycarbonatpolyolen umgearbeitet werden.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin sowie ihre beliebigen Mischungen.

Als Starter können die an sich im Rahmen der Polyesterpolyole genannten mehrwertigen Alkohole einer OH-Funktionalität $\geq 2$ sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden. Besonders bevorzugt sind Propylenoxid-homopolymere sowie statistische oder Block-Copolymere, die Oxyethylen-, Oxypropylen- und / oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen- und Oxybutylen umfasst hierbei alle jeweiligen linearen und verzweigten $C_3$- und $C_4$-Isomere.

Daneben sind als Bestandteile der Polyol-Komponente b1) als polyfunktionelle, isocyanatreaktive Verbindungen auch niedermolekulare, d.h. mit Molekulargewichten $\leq 500$ g/mol, kurzkettige, d.h. 2 bis 20 Kohlenstoffatome enthaltende aliphatische, araliphatische oder cycloaliphatische di-, tri- oder polyfunktionelle Alkohole geeignet.

Dies können beispielsweise in Ergänzung zu den oben genannten Verbindungen Neopentylglykol, 2-Ethyl-2-butylpropandiol, Trimethylpentandiol, stellungs-isomere Diethyloctandiole, Cyclohexandiol, 1,4-Cyclohexandimethanol, 1,6-Hexandiol, 1,2- und 1,4-Cyclohexandiol, hydriertes Bisphenol A, 2,2-Bis(4-hydroxy-cyclohexyl)-propan oder 2,2-Dimethyl-3-hydroxypropionsäure, 2,2-dimethyl-3-hydroxypropyl-ester sein. Beispiele geeigneter Triole sind Trimethylolethan, Trimethylolpropan oder Glycerin. Geeignete höherfunktionelle Alkohole sind Di-(trimethylolpropan), Pentaerythrit, Dipenta-erythrit oder Sorbit.

Besonders bevorzugt ist, wenn die Polyolkomponente ein difunktioneller Polyether-, Polyester oder ein Polyether-polyester-block-copolyester oder ein Polyether-Polyester-Blockcopolymer mit primären OH-Funktionen ist.

Es ist ebenfalls möglich, als isocyanatreaktive Verbindungen b1) Amine einzusetzen. Beispiele geeigneter Amine sind Ethylendiamin, Propylendiamin, Diaminocyclohexan, 4,4'-Dicylohexylmethandiamin, Isophorondiamin (IPDA), difunktionelle Polyamine wie z.B. die Jeffamine®, aminterminierte Polymere, insbesondere mit zahlenmittleren Molmassen $\leq 10000$ g/Mol. Mischungen der vorgenannten Amine können ebenfalls verwendet werden.

Es ist ebenfalls möglich, als isocyanatreaktive Verbindungen b1) Aminoalkohole einzusetzen. Beispiele geeigneter Aminoalkohole sind die isomeren Aminoethanole, die isomere Aminopropanole die isomeren Aminobutanole und die isomeren Aminohexanole oder deren beliebige Mischungen.

Alle vorgenannten isocyanatreaktiven Verbindungen b1) können untereinander beliebig vermischt werden.

Bevorzugt ist auch, wenn die isocyanatreaktiven Verbindungen b1) eine zahlenmittlere Molmasse von ≥ 200 und ≤ 10000 g/Mol, weiter bevorzugt ≥ 500 und ≤ 8000 g/Mol und ganz besonders bevorzugt ≥ 800 und ≤ 5000 g/Mol aufweisen. Die OH-Funktionalität der Polyole beträgt bevorzugt 1.5 bis 6.0, besonders bevorzugt 1.8 bis 4.0.

Die Prepolymere der Polyisocyanat-Komponente a) können insbesondere einen Restgehalt an freiem monomeren Di- und Triisocyanaten < 1 Gew.-%, besonders bevorzugt < 0.5 Gew.-% und ganz besonders bevorzugt < 0.3 Gew.-% aufweisen.

Es ist gegebenenfalls auch möglich, dass die Polyisocyanat-Komponente a) vollständig oder anteilsmäßig organische Verbindung enthält, deren NCO-Gruppen ganz oder teilweise mit aus der Beschichtungstechnologie bekannten Blockierungsmitteln umgesetzt sind. Beispiel für Blockierungsmittel sind Alkohole, Lactame, Oxime, Malonester, Pyrazole sowie Amine, wie z.B. Butanonoxim, Diisopropylamin, Malonsäurediethylester, Acetessigester, 3,5-Dimethylpyrazol, ε-Caprolactam, oder deren Mischungen.

Besonders bevorzugt ist, wenn die Polyisocyanat-Komponente a) Verbindungen mit aliphatisch gebundenen NCO-Gruppen umfasst, wobei unter aliphatisch gebundenen NCO-Gruppen derartige Gruppen verstanden werden, die an ein primäres C-Atom gebunden sind. Die isocyanatreaktive Komponente b) umfasst bevorzugt wenigstens eine organische Verbindung, die im Mittel wenigstens 1.5 und bevorzugt 2 bis 3 isocyanatreaktive Gruppen aufweist. Im Rahmen der vorliegenden Erfindung werden als isocyanatreaktive Gruppen bevorzugt Hydroxy-, Amino- oder Mercapto-Gruppen angesehen.

Die isocyanatreaktive Komponente kann insbesondere Verbindungen umfassen, die im Zahlenmittel wenigstens 1.5 und bevorzugt 2 bis 3 isocyanatreaktive Gruppen aufweisen.

Geeignete polyfunktionelle, isocyanatreaktive Verbindungen der Komponente b) sind beispielsweise die oben beschriebenen Verbindungen b1).

Ganz besonders bevorzugt ist auch, wenn die Polyurethane auf Polyester-C4-Polyetherpolyolen basieren.

Photoinitiatoren der Komponente C) sind üblicherweise durch aktinische Strahlung aktivierbare Verbindungen, die eine Polymerisation der Schreibmonomere auslösen können. Bei den Photoinitiatoren kann zwischen unimolekularen (Typ I) und bimolekularen (Typ II) Initiatoren unterschieden werden. Des Weiteren werden sie je nach ihrer chemischen Natur in Photoinitiatoren für radikalische, anionische, kationische oder gemischte Art der Polymerisation unterschieden.

Typ I-Photoinitiatoren (Norrish-Typ-I) für die radikalische Photopolymerisation bilden beim Bestrahlen durch eine unimolekulare Bindungsspaltung freie Radikale. Beispiele für Typ I-Photoinitiatoren sind Triazine, Oxime, Benzoinether, Benzilketale, Bis-imidazole, Aroylphosphinoxide, Sulfonium- und Iodoniumsalze.

Typ II-Photoinitiatoren (Norrish-Typ-II) für die radikalische Polymerisation bestehen aus einem Farbstoff als Sensibilisator und einem Coinitiator und durchlaufen bei der Bestrahlung mit auf den Farbstoff angepasstem Licht eine bimolekulare Reaktion. Zunächst absorbiert der Farbstoff ein Photon und überträgt aus einem angeregten Zustand Energie auf den Coinitiator. Dieser setzt durch Elektronen- oder Protonentransfer oder direkte Wasserstoffabstraktion die polymerisationsauslösenden Radikale frei.

Im Sinne dieser Erfindung werden bevorzugt Typ II-Photoinitiatoren verwendet.

Solche Photoinitiatorsysteme sind prinzipiell in der EP 0 223 587 A beschriebenen und bestehen bevorzugt aus einer Mischung von einem oder mehreren Farbstoffen mit Ammoniumalkylarylborat(en).

Geeignete Farbstoffe, die zusammen mit einem Ammoniumalkylarylborat einen Typ II-Photoinitiator bilden, sind die in der WO 2012062655 beschriebenen kationischen Farbstoffe in Kombination mit den eben dort beschriebenen Anionen.

Unter kationischen Farbstoffen werden bevorzugt solche der folgenden Klassen verstanden: Acridin-Farbstoffe, Xanthen-Farbstoffe, Thioxanthen-Farbstoffe, Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, extern kationische Merocyanin-Farbstoffe, extern kationische Neutrocyanin-Farbstoffe, Nullmethin-Farbstoffe - insbesondere Naphtholactam-Farbstoffe, Streptocyanin-Farbstoffe. Solche Farbstoffe sind beispielsweise in H. Berneth in Ullmann's Encyclopedia of Industrial Chemistry, Azine Dyes, Wiley-VCH Verlag, 2008, H. Berneth in Ullmann's Encyclopedia of Industrial Chemistry, Methine Dyes and Pigments, Wiley-VCH Verlag, 2008, T. Gessner, U. Mayer in Ullmann's Encyclopedia of Industrial Chemistry, Triarylmethane and Diarylmethane Dyes, Wiley-VCH Verlag, 2000 beschrieben.

Besonders bevorzugt sind Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, Nullmethin-Farbstoffe - insbesondere Naphtholactam-Farbstoffe, Streptocyanin-Farbstoffe.

Beispiele für kationische Farbstoffe sind Astrazon Orange G, Basic Blue 3, Basic Orange 22, Basic Red 13, Basic Violett 7, Methylenblau, Neu Methylenblau, Azur A, 2,4-Diphenyl-6-(4-methoxyphenyl)pyrylium, Safranin O, Astraphloxin, Brilliant Grün, Kristallviolett, Ethylviolett und Thionin.

Bevorzugte Anionen sind insbesondere $C_8$- bis $C_{25}$-Alkansulfonat, vorzugsweise $C_{13}$- bis $C_{25}$-Alkansulfonat, $C_3$- bis $C_{18}$-Perfluoralkansulfonat, $C_4$- bis $C_{18}$-Perfluoralkansulfonat, das in der Alkylkette mindestens 3 Wasserstoffatome trägt, $C_9$- bis $C_{25}$-Alkanoat, $C_9$- bis $C_{25}$-Alkenoat, $C_8$- bis $C_{25}$-Alkylsulfat, vorzugsweise $C_{13}$- bis $C_{25}$-Alkylsulfat, $C_8$- bis $C_{25}$-Alkenylsulfat, vorzugsweise $C_{13}$- bis $C_{25}$-Alkenylsulfat, $C_3$- bis $C_{18}$-Perfluoralkylsulfat, $C_4$-bis $C_{18}$-Perfluoralkylsulfat, das in der Alkylkette mindestens 3 Wasserstoffatome trägt, Polyethersulfate basierend auf mindestens 4 Äquivalenten Ethylenoxid und/oder Äquivalenten 4 Propylenoxid, Bis-$C_4$- bis $C_{25}$-Alkyl-, $C_5$- bis $C_7$-Cycloalkyl-, $C_3$- bis $C_8$-Alkenyl- oder $C_7$-bis $C_{11}$-Aralkyl-sulfosuccinat, durch mindestens 8 Fluoratome substituiertes Bis-$C_2$- bis $C_{10}$-alkylsulfosuccinat, $C_8$- bis $C_{25}$-Alkyl-sulfoacetate, durch mindestens einen Rest der Gruppe Halogen, $C_4$- bis $C_{25}$-Alkyl, Perfluor-$C_1$- bis $C_8$-Alkyl und/oder $C_1$- bis $C_{12}$-Alkoxycarbonyl substituiertes Benzolsulfonat, ggf. durch Nitro, Cyano, Hydroxy, $C_1$- bis $C_{25}$-Alkyl, $C_1$- bis $C_{12}$-Alkoxy, Amino, $C_1$- bis $C_{12}$-Alkoxycarbonyl oder Chlor substituiertes Naphthalin- oder Biphenylsulfonat, ggf. durch Nitro, Cyano, Hydroxy, $C_1$- bis $C_{25}$-Alkyl, $C_1$- bis $C_{12}$-Alkoxy, $C_1$- bis $C_{12}$-Alkoxycarbonyl oder Chlor substituiertes Benzol-, Naphthalin- oder Biphenyldisulfonat, durch Dinitro, $C_6$- bis $C_{25}$-Alkyl, $C_4$- bis $C_{12}$-Alkoxycarbonyl, Benzoyl, Chlorbenzoyl oder Toluoyl substituiertes Benzoat, das Anion der Naphthalindicarbonsäure, Diphenyletherdisulfonat, sulfonierte oder sulfatierte, ggf. mindestens einfach ungesättigte $C_8$- bis $C_{25}$-Fettsäureester von aliphatischen $C_1$- bis $C_8$-Alkoholen oder Glycerin, Bis-(sulfo-$C_2$- bis $C_6$-alkyl)-$C_3$- bis $C_{12}$-alkandicarbonsäureester, Bis-(sulfo-$C_2$- bis $C_6$-alkyl)-itaconsäureester, (Sulfo-$C_2$- bis $C_6$-alkyl)-$C_6$- bis $C_{18}$-alkancarbonsäureester, (Sulfo-$C_2$- bis $C_6$-alkyl)-acryl- oder methacrylsäureester, ggf. durch bis zu 12 Halogenreste substituiertes Triscatecholphosphat, ein Anion der Gruppe Tetraphenylborat, Cyanotriphenylborat, Tetraphenoxyborat, $C_4$- bis $C_{12}$-Alkyl-triphenylborat, deren Phenyl- oder Phenoxy-Reste durch Halogen, $C_1$- bis $C_4$-Alkyl und/oder $C_1$- bis $C_4$-Alkoxy substituiert sein können, $C_4$- bis $C_{12}$-Alkyl-trinaphthylborat, Tetra-$C_1$- bis $C_{20}$-alkoxyborat, 7,8- oder 7,9-Dicarba-nidoundecaborat(1-) oder (2-), die gegebenenfalls an den B- und/oder C-Atomen durch eine oder zwei $C_1$- bis $C_{12}$-Alkyl- oder Phenyl-Gruppen substituiert sind, Dodecahydrodicarbadodecaborat(2-) oder B-$C_1$- bis $C_{12}$-Alkyl-C-phenyl-dodecahydrodicarbadodecaborat(1-) steht, wobei bei mehrwertigen Anionen wie Naphthalindisulfonat $A^-$ für ein Äquivalent dieses Anions steht, und wobei die Alkan- und Alkylgruppen verzweigt sein können und/oder durch Halogen, Cyano, Methoxy, Ethoxy, Methoxycarbonyl oder Ethoxycarbonyl substituiert sein können.

Bevorzugt ist auch, wenn das Anion $A^-$ des Farbstoffs einen AClogP im Bereich von 1 bis 30, besonders bevorzugt im Bereich von 1 bis 12 und insbesondere bevorzugt im Bereich von 1 bis 6,5 aufweist. Der AClogP wird nach J. Comput. Aid. Mol. Des. 2005, 19, 453; Virtual Computational Chemistry Laboratory, http://www.vcclab.org berechnet.

Geeignete Ammoniumalkylarylborate sind beispielsweise (Cunningham et al., RadTech'98 North America UV/EB Conference Proceedings, Chicago, Apr. 19-22, 1998): Tetrabutylammonium Triphenylhexylborat, Tetrabutylammonium Triphenylbutylborat, Tetrabutylammonium Trinapthylhexylborat, Tetrabutylammonium Tris(4-tert.butyl)-phenylbutylborat, Tetrabutylammonium Tris-(3-fluorphenyl)-hexylborat hexylborat ([191726-69-9], CGI 7460, Produkt der BASF SE, Basel, Schweiz), 1-Methyl-3-octylimidazolium Dipentyldiphenylborat und Tetrabutylammonium Tris-(3-chlor-4-methylphenyl)-hexylborat ([1147315-11-4], CGI 909, Produkt der BASF SE, Basel, Schweiz).

Es kann vorteilhaft sein, Gemische dieser Photoinitiatoren einzusetzen. Je nach verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 - 328 beschrieben.

Ganz besonders bevorzugt ist, wenn der Photoinitiator eine Kombination von Farbstoffen, deren Absorptionsspektren zumindest teilweise den Spektralbereich von 400 bis 800 nm abdecken, mit wenigstens einem auf die Farbstoffe abgestimmten Coinitiator umfasst.

Bevorzugt ist auch, wenn wenigstens ein für eine Laserlichtfarbe ausgewählt aus blau, grün und rot geeigneter Photoinitiator in der Photopolymer-Formulierung enthalten ist.

Weiter bevorzugt ist auch, wenn die Photopolymer-Formulierung für wenigstens zwei Laserlichtfarben ausgewählt aus blau, grün und rot je einen geeigneten Photoinitiator enthält.

Ganz besonders bevorzugt ist schließlich, wenn die Photopolymer-Formulierung für jede der Laserlichtfarben blau, grün und rot jeweils einen geeigneten Photoinitiator enthält.

Besonders hohe Brechungsindexkontraste können erzielt werden, wenn die Photopolymer-Formulierung als weiteres Schreibmonomer A) neben dem Schreibmonomer der Formel (I) bevorzugt ein acrylat- oder methacrylatfunktionelles Schreibmonomer umfasst. Besonders bevorzugt sind dabei monofunktionelle Schreibmonomere und insbesondere diejenigen monofunktionellen Urethan(meth)acrylate, die in der US 2010/0036013 A1 beschrieben sind.

Geeignete Acrylat-Schreibmonomere sind insbesondere Verbindungen der allgemeinen Formel (IV)

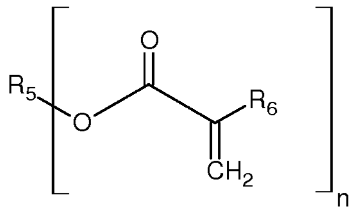

(IV)

bei denen n≥1 und n≤4 ist und $R_5$ ein linearer, verzweigter, cyclischer oder heterocyclischer unsubstituierter oder gegebenenfalls auch mit Heteroatomen substituierter organischer Rest und/oder $R_6$ Wasserstoff, ein linearer, verzweigter, cyclischer oder heterocyclischer unsubstituierter oder gegebenenfalls auch mit Heteroatomen substituierter organischer Rest ist. Besonders bevorzugt ist $R_6$ Wasserstoff oder Methyl und/oder $R_5$ ein linearer, verzweigter, cyclischer oder heterocyclischer unsubstituierter oder gegebenenfalls auch mit Heteroatomen substituierter organischer Rest.

Als Acrylate bzw. Methacrylate werden vorliegend Ester der Acrylsäure bzw. Methacrylsäure bezeichnet. Beispiele bevorzugt verwendbarer Acrylate und Methacrylate sind Phenylacrylat, Phenylmethacrylat, Phenoxyethylacrylat, Phenoxyethylmethacrylat, Phenoxyethoxyethylacrylat, Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Bisphenol A Diacrylat, Bisphenol A Dimethacrylat, sowie deren ethoxylierte Analogverbindungen, N-Carbazolylacrylate.

Als Urethanacrylate werden vorliegend Verbindungen mit mindestens einer Acrylsäureestergruppe und mindestens eine Urethanbindung verstanden. Solche Verbindungen können beispielsweise durch Umsetzung eines Hydroxy-funktionellen Acrylats oder Methacrylats mit einer Isocyanat-funktionellen Verbindung erhalten werden.

Beispiele hierfür verwendbarer Isocyanat-funktionelle Verbindungen sind Monoisocyanate sowie die unter a) genannten monomeren Diisocyanate, Triisocyanate und / oder Polyisocyanate. Beispiele geeigneter Monoisocyanate sind Phenylisocyanat, die isomeren Methylthiophenylisocyanate. Di-, Tri- oder Polyisocyanate sind oben genannt sowie Triphenylmethan-4,4',4''-triisocyanat und Tris(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische Di-, Tri- oder Polyisocyanate.

Als hydroxyfunktionelle Acrylate oder Methacrylate für die Herstellung von Urethanacrylaten kommen beispielsweise Verbindungen wie 2-Hydroxyethyl(meth)acrylat, Polyethylenoxid-mono(meth)acrylate, Polypropylenoxidmono(meth)acrylate, Polyalkylenoxidmono(meth)-acrylate, Po|y(ε-caprolacton)mono(meth)acrylate, wie z.B. Tone® M100 (Dow, Schwalbach, DE), 2-Hydroxypropyl(meth)acrylat, 4-Hydroxybutyl(meth)acrylat, 3-Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylat.

Ebenfalls verwendet werden können die an sich bekannten hydroxylgruppenhaltigen Epoxy-(meth)acrylate mit OH-Gehalten von 20 bis 300 mg KOH/g oder hydroxylgruppenhaltige Polyurethan(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten.

Bevorzugt sind insbesondere Urethanacrylate erhältlich aus der Umsetzung von Tris(p-isocyanatophenyl)thiophosphat und / oder m-Methylthiophenylisocyanat mit alkoholfunktionellen Acrylaten wie Hydroxyethyl(meth)acrylat, Hydroxypropyl(meth)acrylat und / oder Hydroxybutyl(meth)acrylat.

Ebenso ist es möglich, dass das Schreibmonomer weitere ungesättigte Verbindungen wie α,β-ungesättigte Carbonsäurederivate wie beispielsweise Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, α-Methylstyrol, Vinyltoluol und / oder Olefine, umfasst oder daraus besteht.

Gemäß einer weiteren bevorzugten Ausführungsform ist vorgesehen, dass die Photopolymer-Formulierung zusätzlich monomere Fluorurethane umfasst.

Besonders bevorzugt ist, wenn die Fluorurethane wenigstens eine Verbindung der Formel (V)

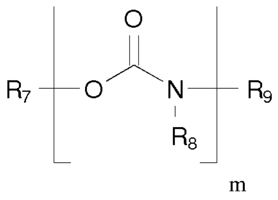

(V)

umfassen oder daraus bestehen, in der m ≥ 1 und m ≤ 8 ist und $R_7$, $R_8$, $R_9$ unabhängig voneinander Wasserstoff, lineare, verzweigte, cyclische oder heterocyclische unsubstituierte oder gegebenenfalls auch mit Heteroatomen substituierte organische Reste sind, wobei bevorzugt mindestens einer der Reste $R_7$, $R_8$, $R_9$ mit wenigstens einem Fluoratom substituiert ist und besonders bevorzugt $R_7$ ein organischer Rest mit mindestens einem Fluoratom ist.

Gemäß einer weiteren bevorzugten Ausführungsform der Erfindung ist vorgesehen, dass die Photopolymer-Formulierung 10 bis 89,999 Gew.-%, bevorzugt 20 bis 70 Gew.-% Matrixpolymere, 3 bis 60 Gew.-%, bevorzugt 10 bis 50 Gew.-% Schreibmonomere, 0,001 bis 5 Gew.-%, bevorzugt 0,5 bis 3 Gew.-% Photoinitiatorenund gegebenenfalls 0 bis 4 Gew.-%, bevorzugt 0 bis 2 Gew.-% Katalysatoren, 0 bis 5 Gew.-% , bevorzugt 0,001 bis 1 Gew.-% Stabilisatoren, 0 bis 40 Gew.-%, bevorzugt 10 bis 30 Gew.-% monomere Fluorurethane und 0 bis 5 Gew.-%, bevorzugt 0,1 bis 5 Gew.-% weitere Additive enthält, wobei die Summe aller Bestandteile 100 Gew.-% beträgt.

Als Katalysatoren können Urethanisierungskatalysatoren, wie z.B. organische oder anorganischen Derivate des Bimuths, des Zinns, des Zinks oder des Eisens (siehe dazu auch die in der 2012/062658 genannten Verbindungen) verwendet werden. Besonders bevorzugte Katalysatoren sind Butylzinn-tris(2-ethylhexanoat), Eisen [III] - tris-acetylacetonat, Bismuth(III) tris(2-ethylhexanoat), und Zinn(II) bis(2-ethylhexanoat). Weiterhin können auch sterisch gehinderte Amine als Katalysatoren eingesetzt werden.

Als Stabilisatoren können

a) Radikalinhibitoren, wie z.B. Phenole wie para-Methoxyphenol, 2,6-Di-tert.-butyl-4-methylphenol (Jonol), sowie oligomere Jonole (z.B. Octadecyl-3-(3,5-di-tert-butyl-4-hydroxyphenyl)propionat), weiterhin sog. HALS-Amine (z.B. N,N'-Bisformyl-N,N'-bis-(2,2,6,6-tetramethyl-4-piperidinyl)-hexamethylendiamin), N-Alkyl-HALS-, N-Alkoxy-HALS- und N-Alkoxyethyl-HALS-Verbindungen sowie Phenothiazin;

b) Antioxidantien, wie z.B. Diphosphonite, Disulfide und Thioether; und / oder

c) UV Absorber, wie Cyanoacrylate (z.B. Ethyl-2-cyano-3,3-diphenylacrylat), Benzotriazole (z.B. 2-(2H-Benzotriazol-2-yl)-4,6-bis(1-methyl-1-phenylethyl)phenol), Benzophenone (z.B. 2-Hydroxy-4-octyloxybenzophenon), Benzotriazole (z.B. 2-(2-hydroxyphenyl)-benzotriazole) und Oxanilide;

zum Einsatz kommen.

Als weitere Additive können

a) Verlaufshilfsmittel (Benetzungshilfsmittel), wie z.B. Polyacrylate, Silikone, Hybridpolymere (z.B. silikonisierte Acrylate), die sich dadurch auszeichnen, daß sie die Oberflächenspannung einstellen, und / oder

b) Antistatika

c) Thixotropiermittel

d) Verdicker und / oder

e) Biozide

eingesetzt werden.

Besonders bevorzugt werden Photopolymer-Formulierungen mit 20 bis 70 Gew.-% Matrixpolymeren, 20 bis 50 Gew.-% Schreibmonomere, 0,001 bis 5 Gew.-% Photoinitiatoren, 0 bis 2 Gew.-% Katalysatoren, 0,001 bis 1 Gew.-% Radikalstabilisatoren gegebenenfalls 10 bis 30 Gew.-% Fluorurethane und gegebenenfalls 0,1 bis 5 Gew.-% weiterer Additiveeingesetzt.

Gegenstand der Erfindung ist auch ein holographisches Medium insbesondere in Form eines Films, enthaltend

eine erfindungsgemäßes Photopolymer-Formulierung oder erhältlich unter Verwendung einer erfindungsgemäßen Photopolymer-Formulierung. Noch ein weiterer Gegenstand der Erfindung ist die Verwendung einer erfindungsgemäßen Photopolymer-Formulierung zur Herstellung holographischer Medien.

Bei einer bevorzugten Ausführungsform des erfindungsgemäßen holographisches Mediums ist in dieses wenigstens ein Hologramm einbelichtet.

Insbesondere kann das Hologramm ein Reflektions-, Transmissions-, In-Line-, Off-Axis-, Full-Aperture Transfer-, Weißlicht-Transmissions-, Denisyuk-, Off-Axis Reflektions- oder Edge-Lit Hologramm sowie ein holographisches Stereogramm und bevorzugt ein Reflektions-, Transmissions- oder Edge-Lit Hologramm sein.

Mögliche optische Funktionen der Hologramme entsprechen den optische Funktionen von Lichtelementen wie Linsen, Spiegel, Umlenkspiegel, Filter, Streuscheiben, gerichteten Streuelementen, Beugungselemente, Lichtleiter, Lichtlenker (waveguides), Projektionsscheiben und/oder Masken. Zudem können mehrere derartiger optischer Funktionen in einem solchen Hologramm kombiniert werden, z.B. so dass je nach Lichteinfall das Licht in eine andere Richtung abgebeugt wird. So kann man beispielsweise mit derartigen Aufbauten autostereoskopische oder holografische elektronische Displays bauen, die es erlauben einen stereoskopischen visuellen Eindruck ohne weitere Hilfsmittel wie z.B. einer Polarisator- oder Shutterbrille zu erleben, zur Verwendung in automobilen Head-up Displays oder Head-mounted Displays.

Häufig zeigen diese optischen Elemente eine spezifische Frequenzselektivität, je nachdem wie die Hologramme belichtet wurden und welche Dimensionen das Hologramm hat. Dies ist insbesondere wichtig, wenn man monochromatische Lichtquellen wie LED oder Laserlicht verwendet. So benötigt man ein Hologramm pro Komplementärfarbe (RGB), um Licht frequenzselektiv zu lenken und gleichzeitig vollfarbige Displays zu ermöglichen. Daher sind in bestimmten Displayaufbauten mehrer Hologramme ineinander im Medium zu belichten.

Zudem können mittels der erfindungsgemäßen Medien auch holographische Bilder oder Darstellungen, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten hergestellt werden. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Designmöglichkeiten stellen Hologramme, insbesondere Volumenhologramme, eine attraktive technische Lösung für die oben genannten Anwendung dar. Auch ist es möglich derartige Hologramme zur Speicherung digitaler Daten zu verwenden, wobei verschiedenste Belichtungsverfahren (Shift-, Spatial- or Angular- Multiplexing) verwendet werden.

Gegenstand der vorliegenden Erfindung ist auch noch ein Verfahren zur Herstellung eines holographischen Mediums unter Verwendung einer erfindungsgemäßen Photopolymer-Formulierung.

So können die Photopolymer-Formulierungen insbesondere zur Herstellung holographischer Medien in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Bevorzugte Materialien oder Materialverbünde des Trägers basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid (PA), Polymethylmethacrylat (PMMA), Polyvinylchlorid, Polyvinylbutyral oder Polydicyclopentadien oder deren Mischungen. Besonders bevorzugt basieren sie auf PC, PET, PA, PMMA und CTA. Materialverbünde können Folienlaminate oder Coextrudate sein. Bevorzugte Materialverbünde sind Duplex- und Triplexfolien aufgebaut nach einem der Schemata A/B, A/B/A oder A/B/C. Besonders bevorzugt sind PC/PET, PET/PC/PET und PC/TPU (TPU = Thermoplastisches Polyurethan).

Die Materialien oder Materialverbünde des Trägers können einseitig oder beidseitig antihaftend, antistatisch, hydrophobiert oder hydrophiliert ausgerüstet sein. Die genannten Modifikationen dienen an der Photopolymerschicht zugewandten Seite dem Zweck, dass die Photopolymerlage von dem Träger zerstörungsfrei abgelöst werden kann. Eine Modifikation der der Photopolymerlage abgewandten Seite des Trägers dient dazu, dass die erfindungsgemäßen Medien speziellen mechanischen Anforderungen genügen, die z.B. bei der Verarbeitung in Rollenlaminatoren, insbesondere bei Rolle-zu-Rolle-Verfahren, gefordert sind.

Ebenfalls Gegenstand der Erfindung ist eine optische Anzeige, umfassend ein erfindungsgemäßes holographisches Medium.

Beispiele für derartige optische Anzeigen sind bildgebende Anzeigen auf Basis von Flüssigkristallen, organischen lichtemittierenden Dioden (OLED), LED-Displaytafeln, Microelektromechanische Systeme (MEMS) auf Basis von diffraktiver Lichtselektion, Electrowettingdisplays (E-ink) und Plasmabildschirmen. Derartige optische Anzeigen können autostereoskopische und/oder holographische Displays, transmittive und reflektive Projektionsleinwände oder Projektionsscheiben, Displays mit schaltbaren eingeschränkten Abstrahlverhalten für Privacyfilter und bidirektionalen Multiuserbildschirmen, virtuelle Bildschirme, Headup-Displays, Head-mounted Displays, Leuchtsymbole, Warnlampen, Signallampen, Scheinwerfer und Schautafeln sein.

Darüber hinaus ist auch die Verwendung eines erfindungsgemäßen holographischen Mediums zur Herstellung von Chipkarten, Ausweisdokumenten, 3D-Bildern, Produktschutzetiketten, Labeln, Banknoten oder holografisch optischen Elementen insbesondere für optische Anzeigen Gegenstand der Erfindung.

Zuletzt ist auch ein aromatischer Glycolether der Formel (I)

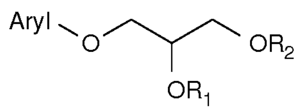
(I)

in der Aryl ein substituierter oder unsubstituierter aromatischer Rest, ausgeschlossen unsubstituiertes Phenyl, ist und $R_1$ und $R_2$ jeweils unabhängig voneinander ein Rest der Formel (II) oder (III)

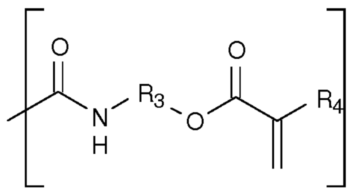
(II)

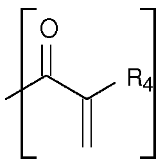
(III)

sind, in denen

$R_3$ ein organischer Rest mit bis zu 6 Kohlenstoffatomen ist, der Sauerstoff- und / oder Schwefelatome enthalten kann,

$R_4$ ein Rest ausgewählt aus der Gruppe -H, $-CH_3$ ist,

wobei Aryl ein Rest ausgewählt aus der Gruppe Methylphenyl, Ethylphenyl, Thiomethylphenyl, Methoxyphenyl, Biphenyl und Naphtyl ist.

Besonders bevorzugt ist hier, wenn jeweils einer der Reste $R_1$ und $R_2$ ein Rest der Formel (II) und jeweils einer der Reste $R_1$ und $R_2$ ein Rest der Formel (III) ist, wobei $R_3$ insbesondere ein $-CH_2CH_2-$ Rest sein kann und noch bevorzugter wobei Rest $R_1$ ein Rest der Formel (III) und der Rest $R_2$ ein Rest der Formel (II) ist.

Bevorzugt ist auch, wenn Rest Aryl mit 1 bis 5 und bevorzugt 1 bis 3 gleichen oder unterschiedlichen Substituenten ausgewählt aus der Gruppe n-Alkyl, verzweigtes Alkyl, Alkyloxy, Phenyl, Benzyl, Phenylalkyl, Naphtyl, Methylthiyl, Ethylthiyl, Alkylthiyl, Alkylthioalkyl, Phenoxy, Phenylthiyl, Napthylthiyl, Fluor, Chlor, Brom und/oder Iod und bevorzugt mit 1 bis 3 gleichen oder unterschiedlichen Substituenten ausgewählt aus der Gruppe Methyl, Ethyl, Thiomethyl, Methoxy, Phenyl substituiert ist.

## Beispiele

Die Erfindung wird im Folgenden anhand von Beispielen näher erläutert.

In der Zeichnung zeigt

Figur 1 die Geometrie eines Holographic Media Testers (HMT) bei $\lambda$ = 532 nm (DPSS Laser = Diode Pumped Solid State Laser) und

Figur 2 die gemessene Beugungseffizienz $\eta$ als Kreise gegen das Winkeldetuning $\Delta\Omega$ aufgetragen und die Anpas-

sung der Kogelnik Theorie als durchgezogene Linie. Die Darstellung zeigt Beispiel 4.4

Figur 3 die gemessene Beugungseffizienz η als Kreise gegen das Winkeldetuning ΔΩ aufgetragen und die Anpassung der Kogelnik Theorie als durchgezogene Linie. Die Darstellung zeigt Beispiel 4.8

**Messmethoden:**

**Viskositätsbestimmung:**

Die Viskosität wurde mit einem Physica MCR 51 (Fa. Anton Paar) Viskosimeter bestimmt, Dazu wurde temperiert und ein Kegel eingehangen (für niedrige Viskositäten η < 10000 mPas: 23°C, 25mm (CP-25) Kegeldurchmesser und für hohe Viskositäten η > 10000 mPas 50°C, 60mm (CP-60) Kegeldurchmesser). Man gab ca. 0,5-1g Produkt auf die Platte, ließ den Kegel herunter fahren, so daß der Kegel mit Produkt vollständig benetzt war. Überstehendes Produkt wurde abgewischt. Die Scherrate (ca. 500 1/s bei niedrigen Viskositäten und ca. 100 1/s bei höheren Viskositäten) wurde von dem Gerät selbständig eingestellt. Es wurden jeweils 20 Messwerte gemessen und der Mittelwert bestimmt.

**Brechungsindexbestimmung:**

Die Messung des Brechungsindex erfolgte für hochviskose und feste Produkte bei einer Wellenlänge von 589 nm indem der Brechungsindex n in Abhängigkeit von der Wellenlänge der Probe aus den Transmissions- und Reflexionsspektren erhalten wurde. Dazu wurden ca. 100 - 300 nm dicke Filme der Proben auf Quarzglasträger aus einer fünf gewichtsprozentigen Lösung in Ethylacetat aufgeschleudert. Das Transmissions- und Reflexionsspektrum dieses Schichtpaketes wurde mit einem Spektrometer der Firma STEAG ETA-Optik, CD-Measurement System ETA-RT gemessen und danach die Schichtdicke und der spektrale Verlauf von n an die gemessenen Transmissions- und Reflexionsspektren angepasst. Dies geschah mit der internen Software des Spektrometers und erforderte zusätzlich die n Daten des Quarzglassubstrates, die in einer Blindmessung vorab bestimmt wurden.

Für flüssige Produkte wurde ein Abbe-Refraktrometer zur Brechungsindexbestimmung bei 589 nm verwendet. Dabei wurden 3 Tropfen des Produktes auf das gesäuberte Meßprisma des Geräts aufgebracht, das Beleuchtungsprisma zugeklappt und innerhalb von 2 Minuten auf 20°C temperiert. Anschließend wurde im Beobachtungsfeld die Hell-Dunkel-Grenze scharf auf das Fadenkreuz des Refraktometers eingestellt. Wenn sich der eingestellte Wert nicht mehr änderte, wurde an der Skala im Gerat die Brechzahl auf vier Stellen nach dem Komma abgelesen. Es wurde eine Doppelbestimmung durchgeführt. Abweichungen bis zu 0,0002 Skalenteile waren zulässig.

**Trübungsmessung**

Die Trübungsmessung erfolgte gemäß ASTM D 1003. Die Trübung stellt den prozentualen Anteil durchgelassenen Lichtes dar, der vom eingestrahlten Lichtbündel im Mittel um mehr als 2.5° abweicht. Zur Messung der Trübung wurden die holografischen Coupons vor der Messung außen gereinigt, um eine Verfälschung des Ergebnisses durch Fingerabdrücke und Schmutz auf den Gläsern zu vermeiden. Dann wurden die Coupons in ein Haze-Gard-Plus Gerät der Byk-Gardner zur Vermessung eingesetzt. Die Schichtdickenmessung des Coupons erfolgt wie unten im Abschnitt "Messung der holographischen Eigenschaften DE und Δn der holographischen Medien mittels Zweistrahlinterferenz in Transmissionsanordnung" beschrieben in der Simulation der theoretischen Braggkurve nach Kogelnik.

**Zeitmessung zur Lösung von Schreibmonomeren**

1,47 g der Polyolkomponente wurden in ein Tablettenröhrchen gefüllt, ein Rührfisch wurde zugegeben und das Tablettenröhrchen wurde auf einen Magnetrührer positioniert. Anschließend wurde unter Rührem 1,00 g des zu prüfenden Schreibmonomers hinzugegeben und die Zeit bestimmt, bis eine visuell klare, homogene Lösung entstanden war.

**Isocyanatgehalt**

Die angegebenen NCO-Werte (Isocyanat-Gehalte) wurden gemäß DIN EN ISO 11909 bestimmt.

Der vollständige Umsatz von NCO-Gruppen bzw. deren Abwesenheit in einem Reaktionsgemisch wurde IR-spektroskopisch nachgewiesen. So wurde dann ein vollständiger Umsatz angenommen, wenn im IR-Spektrum des Reaktionsgemisches keine NCO-Bande (2261$cm^{-1}$) sichtbar war.

**Festkörpergehalt**

Von einem unlackierten Dosendeckel und einer Büroklammer wurde das Tara-Gewicht ermittelt. Dann wurden ca. 1g der zu untersuchenden Probe nach der Einwaage in dem Dosendeckel mit der geeignet gebogenen Büroklammer gleichmäßig verteilt. Die Büroklammer verblieb zur Messung in der Probe. Die Einwaage wurde ermittelt, danach für 1 Stunde bei 125 °C in einen Laborofen erhitzt und anschließend die Auswaage bestimmt. Den Festkörpergehalt wurde gemäß folgender Gleichung bestimmt: Auswaage [g] * 100 / Einwaage [g] = Gew.-% Festkörper.

**Messung der holographischen Eigenschaften DE und $\Delta$n der holographischen Medien mittels Zweistrahlinterferenz in Transmissionsanordnung**

Die hergestellten Medien wurden mittels einer Messanordnung gemäß Figur 1 wie folgt auf ihre holographischen Eigenschaften geprüft:
Figur 1 zeigt den holographischen Versuchsaufbau, mit dem die Beugungseffizienz (DE) der Medien gemessen wurde, wobei folgendes gilt: M = Spiegel, S = Verschluss, SF = Raumfilter, CL = Kollimatorlinse, $\lambda/2$ = $\lambda/2$ Platte, PBS = polarisationsempfindlicher Strahlteiler, D = Detektor, I = Irisblende, $\alpha_0$ = -22.3°, $\beta_0$ = 22.3° sind die Einfallswinkel der kohärenten Strahlen außerhalb der Probe (des Mediums) gemessen. RD = Referenzrichtung des Drehtisches.

Der Strahl eines DPSS Lasers (Emissionswellenlänge 532 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die $\lambda/2$ Plättchen wurden die Leistung des Referenzstrahls auf 2.0 mW und die Leistung des Signalstrahls auf 2.0 mW eingestellt. Die Leistungen wurden mit den Halbleiterdetektoren (D) bei ausgebauter Probe bestimmt. Der Einfallswinkel ($\alpha_0$) des Referenzstrahls beträgt -22.3°, der Einfallswinkel ($\beta_0$) des Signalstrahls beträgt 22.3°. Die Winkel werden ausgehend von der Probennormale zur Strahlrichtung gemessen. Gemäß Figur 1 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die parallel zur Winkelhalbierenden der zwei auf die Probe einfallenden Strahlen liegen (Transmissionshologramm). Der Streifenabstand $\Lambda$, auch Gitterperiode genannt, im Medium beträgt $\sim$ 700 nm (der Brechungsindex des Mediums zu $\sim$1.504 angenommen).

Es wurden auf folgende Weise Hologramme in das Medium geschrieben:

- Beide Shutter (S) sind für die Belichtungszeit *t* geöffnet.
- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega_{min}$ bis $\Omega_{max}$ mit einer Winkelschrittweite von 0.05°. $\Omega$ wird von der Probennormale zur Referenzrichtung des Drehtisches gemessen. Die Referenzrichtung ($\Omega$ = 0) des Drehtisches ergibt sich dann, wenn beim Schreiben des Hologramms der Einfallswinkel des Referenz- und des Signalstrahls betragsmäßig gleich sind also $\alpha_0$ = -22.3° und $\beta_0$ = 22.3° gilt. Allgemein gilt für das Interferenzfeld beim Schreiben ("recording") eines symmetrischen Transmissionshologramms ($\alpha_0$ = - $\beta_0$):

$$\alpha_0 = \theta_0$$

$\theta_0$ ist der Halbwinkel im Laborsystem außerhalb des Mediums. In diesem Fall gilt also $\theta_0$ =-22.3°. An jedem angefahrenen Drehwinkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad η in Abhängigkeit des Drehwinkels Ω des geschriebenen Hologramms gemessen und in einem Computer gespeichert. Zusätzlich wurde auch die in die nullte Ordnung transmittierte Intensität gegen den Drehwinkel Ω aufgezeichnet und in einem Computer gespeichert.

Die zentrale Beugungseffizienz (DE = η0) des Hologramms, wurde bei Ω = 0 ermittelt.

Der Brechungsindexkontrast Δn und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve angepasst. Das Auswerteverfahren wird im Folgenden beschrieben:
Für die Braggkurve η(Ω) eines Transmissionshologramms gilt nach Kogelnik:

$$\eta = \frac{\sin^2\left(\sqrt{\nu^2 + \xi^2}\right)}{1 + \frac{\xi^2}{\nu^2}}$$

mit:

$$\nu = \frac{\pi \cdot \Delta n \cdot d}{\lambda \cdot \sqrt{|c_s \cdot c_r|}}$$

$$\xi = -\frac{d}{2 \cdot c_s} \cdot DP$$

$$c_s = \cos(\vartheta)$$

$$c_r = \cos(\vartheta)$$

$$DP = \frac{\pi}{\Lambda} \cdot \left(-2 \cdot \sin(\vartheta) - \frac{\lambda}{n \cdot \Lambda}\right)$$

$$\Lambda = -\frac{\lambda}{2 \cdot n \cdot \sin(\alpha)}$$

Beim Auslesen des Hologramms ("reconstruction") gilt wie analog oben dargestellt:

$$\vartheta_0 = \theta_0 + \Omega$$

$$\sin(\vartheta_0) = n \cdot \sin(\vartheta)$$

An der Bragg-Bedingung ist das "Dephasing" DP = 0. Und es folgt entsprechend:

$$\alpha_0 = \theta_0$$

$$\sin(\alpha_0) = n \cdot \sin(\alpha)$$

ν ist die Gitterstärke und ξ ist der Detuning Parameter des Brechungsindexgitters das geschrieben wurde. n ist der mittlere Brechungsindex des Photopolymers und wurde zu 1.504 gesetzt. λ ist die Wellenlänge des Laserlichts

im Vakuum.

Die zentrale Beugungseffizienz (DE = $\eta 0$) ergibt sich dann für $\xi = 0$ zu:

$$DE = \sin^2(\nu) = \sin^2\left(\frac{\pi \cdot \Delta n \cdot d}{\lambda \cdot \cos(\alpha)}\right)$$

Die Messdaten der Beugungseffizienz und die theoretische Braggkurve werden wie in Figur 2 und Figur 3 gezeigt gegen den Drehwinkel $\Omega$ aufgetragen.

Da DE bekannt ist wird die Form der theoretischen Braggkurve nach Kogelnik nur noch durch die Dicke *d* der Photopolymerschicht bestimmt. $\Delta n$ wird über DE für gegebene Dicke *d* so nachkorrigiert, dass Messung und Theorie von DE immer übereinstimmen. *d* wird nun solange angepasst bis die Winkelpositionen der ersten Nebenminima und die Höhen der ersten Nebenmaxima der theoretischen Braggkurve mit den Winkelpositionen der ersten Nebenminima und den Höhen der ersten Nebenmaxima der gemessenen Braggkurve übereinstimmen.

Figur 2 und Figur 3 zeigen die theoretisch berechneten und an die experimentellen Daten gefittete Braggkurven $\eta$ nach der Coupled Wave Theorie (auch Kogelnik-Theorie genannt) als durchgezogene Linie und zeigen vergleichend den experimentell bestimmten Beugungswirkungsgrad (in Kreissymbolen) aufgetragen gegen den Drehwinkel $\Omega$.

Für eine Formulierung wurde diese Prozedur eventuell mehrfach für verschiedene Belichtungszeiten t an verschiedenen Medien wiederholt, um festzustellen bei welcher mittleren Energiedosis des einfallenden Laserstrahls beim Schreiben des Hologramms $\Delta n$ in den Sättigungswert übergeht. Die mittlere Energiedosis *E* ergibt sich wie folgt aus den Leistungen der zwei den Winkeln $\alpha_0$ und $\beta_0$ zugeordneten Teilstrahlen (Referenzstrahl mit $P_r$ = 2.00 mW und Signalstrahl mit $P_s$ = 2.00 mW), der Belichtungszeit t und dem Durchmesser der Irisblende (0.4 cm):

$$E\,(\mathrm{mJ/cm^2}) = \frac{2 \cdot [P_r + P_s] \cdot t\,(\mathrm{s})}{\pi \cdot 0.4^2\ \mathrm{cm}^2}$$

**Chemikalien:**

In eckigen Klammern ist jeweils, soweit bekannt die CAS-Nummer angegeben.

| | |
|---|---|
| m-Kresol | [108-39-4] - ABCR GmbH & Co KG, Karlsruhe, Deutschland |
| 3-Ethylphenol | [620-17-7] - Fluka / Sigma-Aldrich Chemie GmbH, Steinheim, Deutschland |
| 3-(Methylthio)phenol | [1073-29-6] - ABCR GmbH & Co KG, Karlsruhe, Deutschland |
| 4-(Methylthio)phenol | [1073-72-9] - Sigma-Aldrich Chemie GmbH, Steinheim, Deutschland |
| Kaliumcarbonat | Sigma-Aldrich Chemie GmbH, Steinheim, Deutschland |
| Epibromhydrin | [3132-64-7]- Sigma-Aldrich Chemie GmbH, Steinheim, Deutschland |
| Phenylglycidether | Denacol EX141; Nagase ChemteX Corporation, Osaka, Japan |
| Triphenylphosphin | [603-35-0] ABCR GmbH & Co KG, Karlsruhe, Deutschland |
| Acrylsäure | [79-10-7] Acros Organics, Geel, Belgium |
| Methacrylsäure | [79-41-4] Acros Organics, Geel, Belgium |
| Jonol | [128-37-0] Merck KGaA, Darmstadt, Germany |
| 2-[(Biphenyl-2-yloxy)methyl]oxiran | Denacol EX142; Nagase ChemteX Corporation, Osaka, Japan |
| 2-[(2-Methylphenoxy)methyl]oxiran | [2210-79-9] Sigma-Aldrich Chemie GmbH, Steinheim, Deutschland |
| 2-Isocyanatoethylacrylat | [13641-96-8] - Karenz® AOI, SHOWA DENKO K.K., Fine Chemicals Group, Specialty Chemicals Department, Chemicals Division, Japan |
| 2-Isocyanatoethylmethacrylat | [30674-80-7] - Karenz® MOI, SHOWA DENKO K.K., Fine Chemicals Group, Specialty Chemicals Department, Chemicals Division, Japan |

(fortgesetzt)

| | |
|---|---|
| 1,2-Cyclohexanamino-N,N'-bis (3,5-Di-t.-butylsalicyl-idene)cobalt(III)-p-toluolsulfonat monohydrat | [672306-06-8] ABCR GmbH & Co KG, Karlsruhe, Deutschland |
| 1-Isocyanato-3-(methylsulfanyl)benzol | [28479-19-8]- Sigma-Aldrich Chemie GmbH, Steinheim, Deutschland |
| Tris(p-isocyanatophenyl)thiophosphat | Desmodur® RFE, Produkt der Bayer MaterialScience AG, Leverkusen, Deutschland |
| Dibutylzinndilaurat | [77-58-7] - Urethanisierungskatalysator Desmorapid Z, Bayer MaterialScience AG, Leverkusen, Deutschland |
| Fomrez® UL 28 | Urethanisierungskatalysator, Handelsprodukt der Momentive Performance Chemicals, Wilton, CT, USA. |
| Addocat® SO | ein Zinn basierender Katalysator der Rhein-Chemie, Mannheim, Deutschland |
| Desmodur® N 3900 | Produkt der Bayer MaterialScience AG, Leverkusen, DE, Hexandiisocyanatbasiertes Polyisocyanat, Anteil an Iminooxadiazindion mindestens 30 %, NCO-Gehalt: 23.5 %. |
| CGI-909 | Tetrabutylammonium-tris(3-chlor-4-methylphenyl)(hexyl)borat, [1147315-11-4] ist ein Produkt der BASF SE (ehem. Ciba Inc.). |
| Trimethylhexamethylendiisocyanat | [28679-16-5] - ABCR GmbH & Co KG, Karlsruhe, Deutschland |
| 1H,1H-7H-Perfluorheptan-1-ol | [335-99-9] - ABCR GmbH & Co KG, Karlsruhe, Deutschland |
| Kristallviolett | [548-62-9] Sigma-Aldrich Chemie GmbH, Steinheim, Deutschland |
| Irgacure® 250 | [344562-80-7], Iodonium, (4-methylphenyl)[4-(2-methylpropyl) phenyl]-, hexafluorophosphate(1-) Produkt der BASF SE |

**Erfindungsgemäße Schreibmonomere:**

**Allgemeine Vorschrift zur Herstellung der Oxirane (Beispiele 1.5 bis 1.7, andere verwendete Oxirane sind kommerziell erhältlich)**

1 Equivalent Phenol und 2,4 Equivalente Kaliumcarbonat wurden in 2-Butanon vorgelegt. Dann wurden 3 Equivalente Epibromhydrin bei Raumtemperatur langsam zugegeben. Die Menge an 2-Butanon entsprach dabei 50 Gewichtsprozent der Gesamtmenge. Es wurde vorgeprüft, ob sich das Phenol in 2-Butanon hinreichend löst. Die Kaliumcarbonatsuspension wurde dann unter Rückfluß gekocht.

Nachdem vollständiger Umsatz erreicht worden war, der durch $^1$H-NMR Spektroskopie geprüft wurde (Zeitangabe siehe unten), wurde das Kaliumcarbonat abfiltriert, und am Rotationsverdampfer eingeengt. Dabei wurden die flüssigen, klaren, teilweise gefärbten Produkte ohne weitere Aufarbeitung erhalten. Dis Ausbeute bezüglich des eingesetzten Phenols war quantitativ.

| | |
|---|---|
| **Beispiel 1.5** | 2-[(3-Methylphenoxy)methyl]oxiran |
| Edukte: | 11,9 g m-Kresol |
| | 45,2 g Epibromhydrin |
| | 36,4 g Kaliumcarbonat |
| | 93,5 g 2-Butanon |
| Bedingungen: | 16,5 h bei 86 °C und 49,5 Stunden bei 70°C |
| $^1$H-NMR | ($CDCl_3$, 400 MHz): $\delta$ (1H) = 7,18 (t, 1H), 6,77 (d, 1H), 6,68- 6,75 (m, 2H), 4,18 (dd, 1H), 3,95 (dd, 1H), 3,24 (m, 1H), 2,89 (dd, 1H), 2,73 (dd, 1H), 2,32 (s, 3H). |

| | |
|---|---|
| **Beispiel 1.6** | 2-[(3-Ethylphenoxy)methyl]oxiran |
| Edukte: | 12,2 g 3-Ethylphenol |
| | 41,1 g Epibromhydrin |
| | 33,1 g Kaliumcarbonat |
| | 86,4 g 2-Butanon |

(fortgesetzt)

| | |
|---|---|
| **Beispiel 1.6** | 2-[(3-Ethylphenoxy)methyl]oxiran |
| Bedingungen: | 19,3 h bei 86 °C und 50 Stunden bei 70°C |
| $^1$H-NMR | ($CDCl_3$, 400 MHz): $\delta$ (1H) = 7,40 (t, 1H), 6,80 (dd, 1H), 6,77 (m, 2H), 6,70 (dd, 1H), 4,40 (dd, 1H), 3,95 (dd, 1H), 3,35 (m, 1H), 2,85 (dd, 1H), 2,75 (dd, 1H), 2,65 (q, 2H), 1,25 (t, 3H). |

| | |
|---|---|
| **Beispiel 1.7** | 2-{[4-(Methylsulfanyl)phenoxy]methyl}oxiran |
| Edukte: | 15,4 g 4-(Methylthio)phenol |
| | 45,2 g Epibromhydrin |
| | 36,4 g Kaliumcarbonat |
| | 97,1 g 2-Butanon |
| Bedingungen: | 16,3 h bei 86 °C und 50,2 Stunden bei 70°C |
| $^1$H-NMR | ($CDCl_3$, 400 MHz): $\delta$ (1H) = 7,25 (AA'BB', 2H), 6,88 (AA'BB', 2H), 4,20 (dd, 1H), 3,90 (dd, 1H), 3,40 (m, 1H), 2,85 (dd, 1H), 2,73 (dd, 1H), 2,40 (s, 3H). |

**Allgemeine Vorschrift zur Herstellung der (Meth)acrylsäure - Oxiran Addukte (Beispiele 2.1- 2.11)**

Das Oxiran, der Katalysator, Stabilisator und die (Meth)acrylsäure wurden in einem Dreihalskolben vorgelegt, der mit KPG-Rührer und Rührmotor sowie Trockenrohr ausgerüstet war. Es wurde auf 90°C temperiert und bei dieser Temperatur weitergerührt, bis im $^1$H-NMR-Spektrum ein Umsatz der Oxirangruppe >95% erkennbar war bzw. keine Oxirangruppen mehr nachweisbar waren. (HK=Hauptkomponenten, NK=Nebenkomponente)

| | |
|---|---|
| **Beispiel 2.1+2.2** | Mischung aus 2-Hydroxy-3-phenoxypropylacrylat und 1-Hydroxy-3-phenoxypropan-2-ylacrylat (ca. 85:15) |
| Edukte: | 75,3g Phenylglycidether (Denacol EX141) |
| | 328 mg Triphenylphosphin |
| | 36,0 Acrylsäure |
| | 1,1 mg Jonol |
| Bedingungen: | Reaktionszeit 37 Stunden |
| | Es wurde eine klare, farblose, viskose Flüssigkeit erhalten. |
| $^1$H-NMR | ($CDCl_3$, 400 MHz): $\delta$ (1H)= 7,23-7,32 (m, 2H), 6,92-7,00 (m, 1H), 6,84-6,92 (m, 2H), 6,46 (d, 1H), 6,16 (dd, 1H), 5,97 (d, 1H), 5,28 (p, 1H der NK), 3,9-4,45 (m, 5H), 2,72 (t, 1H der NK, OH), 2,60 (s, breit, 1H, OH). |

| | |
|---|---|
| **Beispiel 2.3** | Mischung aus 2-Hydroxy-3-phenoxypropylmethacrylat und 1-Hydroxy-3-phenoxypropan-2-ylmethacrylat (ca. 85:15) |
| Edukte: | 37,7 g Phenylglycidether (Denacol EX141) |
| | 164 mg Triphenylphosphin |
| | 21,5 g Methacrylsäure |
| | 0,6 mg Jonol |
| Bedingungen: | Reaktionszeit 34,5 Stunden |
| | Es wurde eine klare, farblose, viskose Flüssigkeit erhalten. |
| $^1$H-NMR | ($CDCl_3$, 400 MHz): $\delta$ (1H)= 7,25-7,32 (m, 2H), 6,96 (t, 1H), 6,91 (d, 2H), 6,14 (s, 1H), 5,60 (s, 1H), 5,28 (p, 1H der NK), 3,35 (m, 2H der HK), 4,29 (p, 1H der HK), 4,20 (d, 2H der NK), 4,0-4,1 (m, 2H der HK), 3,94 (m, 2H der NK), 2,69 (s, breit, 1H, OH), 1,95 (s, 3H). |

| | |
|---|---|
| **Beispiel 2.4** | Mischung aus 2-Hydroxy-3-(2-methylphenoxy)propylacrylat und 1-Hydroxy-3-(2-methylphenoxy)propan-2-ylacrylat (ca. 85:15) |
| Edukte: | 16,4g 2-[(2-Methylphenoxy)methyl]oxiran |
| | 66 mg Triphenylphosphin |
| | 7,2g Acrylsäure |
| | 11,8 mg Jonol |
| Bedingungen: | Reaktionszeit 17 Stunden |

(fortgesetzt)

| | |
|---|---|
| **Beispiel 2.4** | Mischung aus 2-Hydroxy-3-(2-methylphenoxy)propylacrylat und 1-Hydroxy-3-(2-methylphenoxy)propan-2-ylacrylat (ca. 85:15) |
| | Es wurde eine klare, farblose, viskose Flüssigkeit erhalten. |
| $^1$H-NMR | ($CDCl_3$, 400 MHz): $\delta$ (1H)= 7,1-7,2 (m, 2H), 6,88 (t, 1H), 6,80 (d, 1H), 6,44 (d, 1H), 6,18 (dd, 1H), 5,88 (d, 1H), 5,32 (p, 1H der NK), 4,35-4,45 (m, 2H der HK), 4,29 (p, 1H der HK), 4,19 (d, 2H der NK), 4,0-4,1 (m, 2H der HK), 3,96 (m, 2H der NK), 2,93 (s, breit, 1H, OH), 2,22 (s, 3H der HK), 2,20 (s, 3H, der NK). |
| **Beispiel 2.5** | Mischung aus 2-Hydroxy-3-(3-methylphenoxy)propylacrylat und 1-Hydroxy-3-(3-methylphenoxy)propan-2-ylacrylat (ca. 85:15) |
| Edukte: | 16,5g Beispiel 1.5 |
| | 66 mg Triphenylphosphin |
| | 7,3g Acrylsäure |
| | 11,9 mg Jonol |
| Bedingungen: | Reaktionszeit 17 Stunden |
| | Es wurde eine klare, farblose, viskose Flüssigkeit erhalten. |
| $^1$H-NMR | ($CDCl_3$, 400 MHz): $\delta$ (1H)= 7,13-7,20 (t, 1H), 6,78 (d, 1H), 6,68-6,75 (m, 2H), 6,45 (d, 1H), 6,18 (dd, 1H), 5,87 (d, 1H), 5,28 (p, 1H der NK), 4,3-4,4 (m, 2H der HK), 4,28 (p, 1H der HK), 4,15-4,25 (m, 2H der NK), 3,98-4,08 (m, 2H der HK), 3,94 (m, 2H der NK), 2,90 (s, breit, 1H, OH), 2,30 (s, 3H). |
| **Beispiel 2.6** | Mischung aus 2-Hydroxy-3-(3-ethylphenoxy)propylacrylat und 1-Hydroxy-3-(3-ethylphenoxy) propan-2-ylacrylat (ca. 85:15) |
| Edukte: | 15,3g Beispiel 1.6 |
| | 56 mg Triphenylphosphin |
| | 6,2g Acrylsäure |
| | 10,8 mg Jonol |
| Bedingungen: | Reaktionszeit 17 Stunden |
| | Es wurde eine klare, farblose, viskose Flüssigkeit erhalten. |
| $^1$H-NMR | ($CDCl_3$, 400 MHz): $\delta$ (1H)= 7,13-7,20 (m, 1H), 6,80 (d, 1H), 6,75 (s, 1H), 6,71 (dd, 1H), 6,45 (d, 1H), 6,18 (dd, 1H), 5,85 (d, 1H), 5,28 (p, 1H der NK), 4,3-4,4 (m, 2H der HK), 4,25 (p, 1H der HK), 4,19 (d, 2H der NK), 3,99-4,07 (m, 2H der HK), 3,93 (d, 2H der NK), 3,35 (s, breit, 1H, OH), 2,60 (q, 2H), 1,21 (t, 3H). |
| **Beispiel 2.7** | Mischung aus 2-Hydroxy-3-[4-(methylsulfanyl)phenoxy]-propylacrylat und 1-Hydroxy-3-[4-(methylsulfanyl)phenoxy]propan-2-ylacrylat (ca. 85:15) |
| Edukte: | 35,7g Beispiel 1.7 |
| | 119 mg Triphenylphosphin |
| | 13,1g Acrylsäure |
| | 24,4 mg Jonol |
| Bedingungen: | Reaktionszeit 39 Stunden bei 70°C |
| GC-MS (EI) | Retentionszeit (aus Acetonitril Lösung, es wurden nur alle Nebenkomponenten genannt, die > 10% relativ zur Hauptkomponente im GC erscheinen): |
| | 1. 100%: 17,59 min (MS-EI: m/e = 55, 125, 129, 140, 268) : Isomere Hauptprodukte (M=268) |
| | 2. 17,8%: 3,03 min (MS-EI: m/e= 27, 45, 55, 72) : Acrylsäure (M=72) |
| | 3. 11,2%: 21,30 min (MS-EI: m/e = 55, 57, 115, 129, 353, 382) : 3-({4-[3-(Acryloyloxy)-2-hydroxypropoxy]phenyl}sulfanyl)-2-hydroxypropylacrylat (M= 382) |
| **Beispiel 2.8** | Mischung aus 3-(Biphenyl-2-yloxy)-2-hydroxypropylacrylat und 1-(Biphenyl-2-yloxy)-3-hydroxypropan-2-ylacrylat (ca. 85:15) |
| Edukte: | 191,2 g 2-[(Biphenyl-2-yloxy)methyl]oxiran (Denacol EX 142) |
| | 0,525 g Triphenylphosphin |
| | 57,6 g Acrylsäure |
| | 2,5 mg Jonol |

(fortgesetzt)

| | |
|---|---|
| **Beispiel 2.8** | Mischung aus 3-(Biphenyl-2-yloxy)-2-hydroxypropylacrylat und 1-(Biphenyl-2-yloxy)-3-hydroxypropan-2-ylacrylat (ca. 85:15) |
| Bedingungen: | Reaktionszeit 24,5 Stunden bei 90°C |
| | Es wurde eine klare, farblose, viskose Flüssigkeit erhalten. |
| $^1$H-NMR | ($CDCl_3$, 400 MHz): δ (1H)= 7,45 (d, 2H), 7,38 (t, 2H), 7,2-7,35 (m, 3H), 7,15 (t, 2H), 6,96 (d, 2H), 6,37 (dd, 1H), 6,13 (dd, 1H), 5,81 (d, 1H), 5,28 (p, 1H der NK), 4,37 (t, 1H der NK, OH), 4,28-4,35 (m, 2H), 4,15 (m, 2H der HK), 4,13 (p, 1H), 3,95-4,06 (m, 2H), 3,75 (m, 2H der NK), 2,74 (s, breit, 1H, OH). |
| GC-MS | 1,1% Acrylsäure, 7,8% 2-[(Biphenyl-2-yloxy)methyl]oxiran (Edukt), 1,5% Edukt+HCl, 2,7% Edukt+H2O, 73,2% Produkte(Isomere), 2,2% Produkt+Acrylsäure, 2,7% Edukt+Biphenylphenol, 3,3% Produkt+Edukt |
| **Beispiel 2.9** | Mischung aus 2-Hydroxy-3-(1-naphthyloxy)propylacrylat und 1-Hydroxy-3-(1-naphthyloxy) propan-2-ylacrylat (ca. 85:15) |
| Edukte: | 46,0 g 2-[(1-Naphthyloxy)methyl]oxiran |
| | 0,151 g Triphenylphosphin |
| | 16,6 g Acrylsäure |
| | 0,6 mg Jonol |
| Bedingungen: | Reaktionszeit 22,7 Stunden Es wurde eine klare, farblose, viskose Flüssigkeit erhalten. |
| $^1$H-NMR | ($CDCl_3$, 400 MHz): δ (1H)= 8,18-8,25 (m, 1H), 7,73-7,80 (m, 1H), 7,38-7,49 (m, 3H), 7,29-7,35 (2x t, 1H), 6,76 (d, 1H), 6,44 (2x d, 1H), 6,15 (2x dd, 1H), 5,82 (d, 1H), 5,43 (p, 1H der NK), 4,35-4,50 (m, 3H der HK), 4,30 (d, 2H der NK), 4,13-4,18 (m, 2H der HK), 3,98 (d, 2H der NK), 3,32 (s, breit, 1H, OH), 2,70 (t, 1H der NK, OH). |
| **Beispiel 2.10** | Mischung aus 2-Hydroxy-3-(2-methoxyphenoxy)propylacrylat und 1-Hydroxy-3-(2-methoxyphenoxy)propan-2-ylacrylat (ca. 85:15) |
| Edukte: | 10,8 g 1,2-Epoxy-3-(2-methoxyphenoxy)propan |
| | 0,039 g Triphenylphosphin |
| | 4,3 g Acrylsäure |
| | 7,6 mg Jonol |
| Bedingungen: | Reaktionszeit 29,3 Stunden |
| | Es wurde eine klare, farblose, viskose Flüssigkeit erhalten. |
| $^1$H-NMR | ($CDCl_3$, 400 MHz): δ (1H)= 6,82-7,01 (m, 4H), 6,44 (2x d, 1H), 6,15 (2x dd, 1H), 5,82 (d, 1H), 5,28 (p, 1H der NK), 4,30-4,42 (m, 2H der HK), 4,27 (m, 1H der HK), 4,12 (dd, 1H der HK, ABX), 4,04 (dd, 1H der HK, ABX), 3,98 (d, 2H der NK), 3,78-3,85 (m, 3H, O-Me der HK und der NK + 2H der NK), 3,21 (s, breit, 1H, OH). |
| **Beispiel 2.11** | 2-Hydroxy-3-phenoxypropylacrylat |
| Edukte: | 8,3 g Phenylglycidether |
| | 0,030 g 1,2-Cyclohexanamino-N,N'-bis (3,5-Di-t.-butylsalicylidene)cobalt(III)-p-toluolsulfonat monohydrat |
| | 3,9 g Acrylsäure |
| | 0,1 mg Jonol |
| Bedingungen: | Reaktionszeit 40 Stunden bei Raumtemperatur |
| Aufarbeitung: | 5g des Rohprodukts wurden mit 25g einer Mischung aus Butylacetat und Toluol verdünnt und dann per Schwerkraftsäule von dem Cobalt-Katalysator befreit und unter Zugabe von 0,005mg Jonol im Rotationsverdampfer von dem Lösemittelgemisch befreit. |
| $^1$H-NMR | ($CDCl_3$, 400 MHz): δ (1H) = 7,28 (m, 2H), 6,98 (tt, 1H), 6,92 (dd, 2H), 6,46 (dd, 1H), 6,17 (dd, 1H), 5,87 (dd, 1H), 4,32-4,42 (m, 2H), 4,28 (m, 1H), 4,00-4,09 (m, 2H), 2,65 (d, 1H, OH). Reinheit laut NMR > 92%. |

**Allgemeine Vorschrift zur Herstellung der erfindungsgemäßen aromatischen Glycolether Schreibmonomere (Beispiele 3.1-3.11)**

Die Vorstufe (Beispiel 2.1-2.11), Dibutylzinndilaurat und 2,6-Di-tert.-butyl-4-methylphenol wurden in einem Dreihalskolben vorgelegt, der mit KPG-Rührer und Rührmotor, Gaseinlass sowie Trockenrohr ausgerüstet war. Anschließend wurde auf 60 °C temperiert, langsam Luft übergeleitet und das 2-Isocyanatoethyl(meth)acrylat innerhalb ca. einer halben Stunde unter Rühren zugetropft. Es wurde weiter gerührt bis im IR-Spektrum keine NCO-Bande (2261cm$^{-1}$) mehr beobachtet werden konnte. Die Tabelle 1 zeigt Details der Reaktionsbedingungen und die Charakterisierung der erfindungsgemäßen Schreibmonomere:

Tabelle 1: Herstellungsbedingungen und Charakterisierungen der erfindungsgemäßen Schreibmonomere

| Beispiel | Edukt 1 | Edukt 2 | DBTL | Jonol | Temp | Reaktionszeit | Viskosität | Produktaussehen | Fingerprint (IR, stark, cm-1) |
|---|---|---|---|---|---|---|---|---|---|
| 3.1 | 33,4 g Beispiel 2.1 | 21,2 g AOI | 27 mg | 27 mg | 60°C | 4,5 h | 6350 mPas | klar, gelblich | 984, 888, 810, 757, 693 |
| 3.2 | 33,4 g Beispiel 2.2 | 23,3 g MOI | 28 mg | 28 mg | 60°C | 3,7 h | 4630 mPas | klar, gelb | 985, 948, 887, 811, 756, 693 |
| 3.3 | 58,2 g Beispiel 2.3 | 38,8 g MOI | 49 mg | 49 mg | 60°C | 4,7 h | 3830 mPas | klar, orange-braun | 945, 887, 815, 756, 693 |
| 3.4 | 23,6 g Beispiel 2.4 | 14,1 g AOI | 19 mg | 19 mg | 60°C | 9,2 h | 8790 mPas | Klar, farblos | 983, 808, 751 |
| 3.5 | 23,6 g Beispiel 2.5 | 14,1 g AOI | 19 mg | 19 mg | 60°C | 15,5 h | 5150 mPas | Klar, bernsteinfarben | 989, 809, 777 |
| 3.6 | 21,5 g Beispiel 2.6 | 12,1 g AOI | 17 mg | 17 mg | 60°C | 15,5 h | 2670 mPas | Klar, bernsteinfarben | 984, 814, 778 |
| 3.7 | 25,2 g Beispiel 2.7 | 13,3 g AOI | 19 mg | 19 mg | 60°C | 19,5 h | 174700 mPas | klar, gelblich | 987, 812, 775 |
| 3.8 | 140,7 g Beispiel 2.8 | 62,6 g AOI | 102 mg | 102 mg | 60°C | 13,5 h | 240000 mPas | Klar, farblos | 984, 826, 809, 756, 734, 701, 667 |
| 3.9 | 54,5 g Beispiel 2.9 | 28,2 g AOI | - | - | 60°C | 21,5 h | 176000 mPas | Klar, rotbraun | 984, 809, 794, 773, 738 |
| 3.10 | 15,1 g Beispiel 2.10 | 8,4 g AOI | 12 mg | 12 mg | 60°C | 14 h | 13190 mPas | Klar, farblos | 988, 811 |
| 3.11 | 12,2 g Beispiel 2.11 | 7,7 g AOI | 10 mg | 10 mg | 60°C | 10,3 h | 2640 mPas | Klar, farblos | 978, 814, 756 |

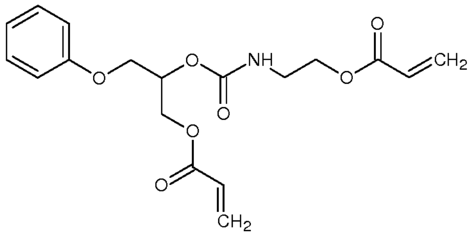 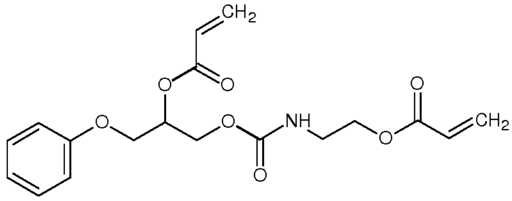

Beispiel 3.1

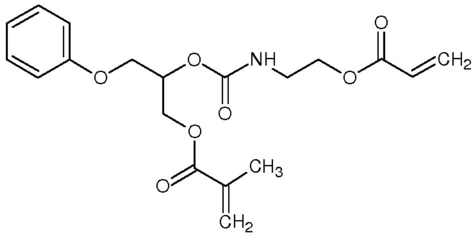 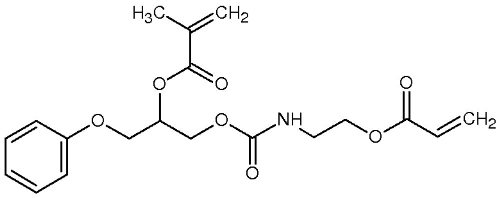

Beispiel 3.2

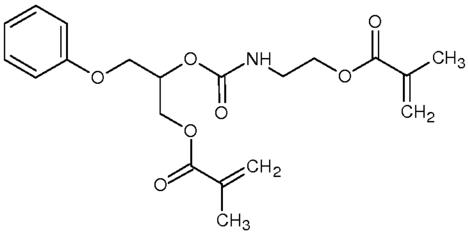 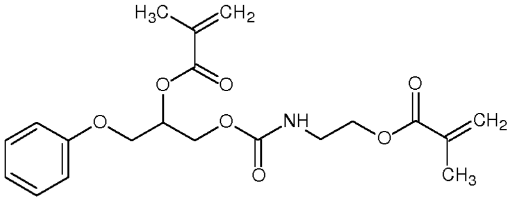

Beispiel 3.3

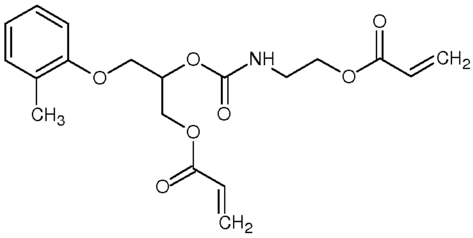 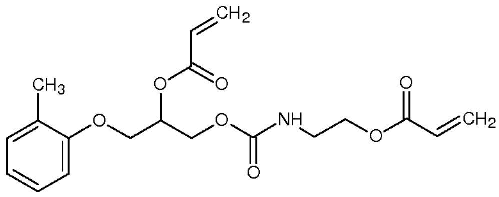

Beispiel 3.4

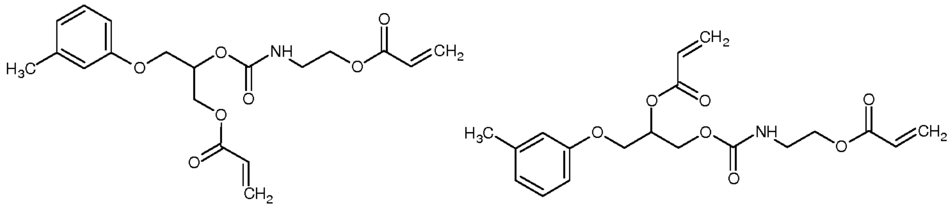

Beispiel 3.5

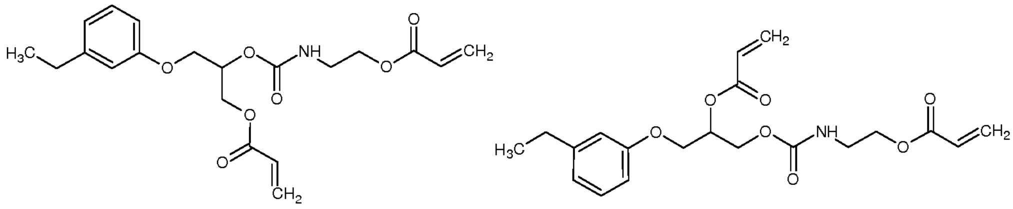

Beispiel 3.6

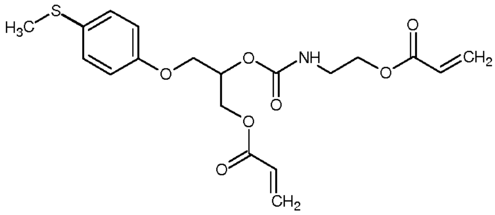

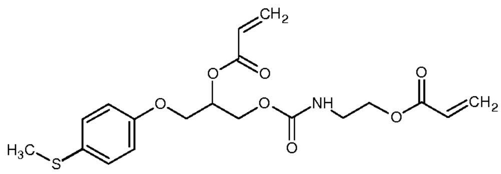

Beispiel 3.7

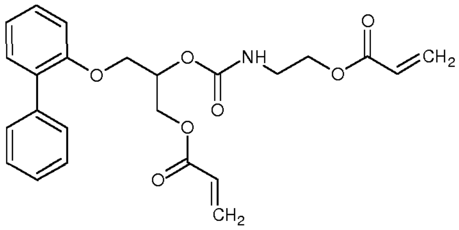

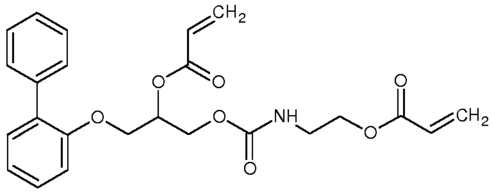

Beispiel 3.8

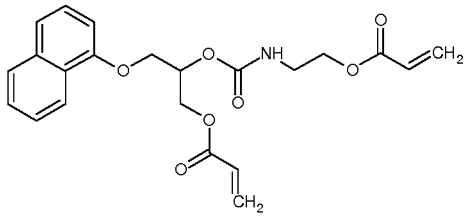

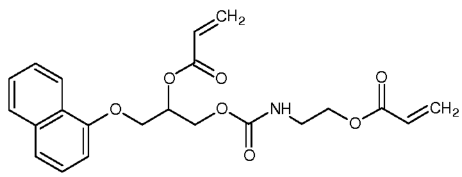

Beispiel 3.9

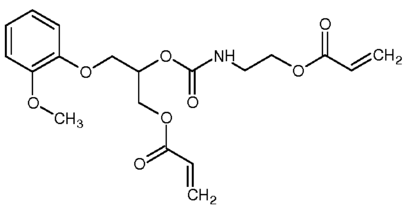

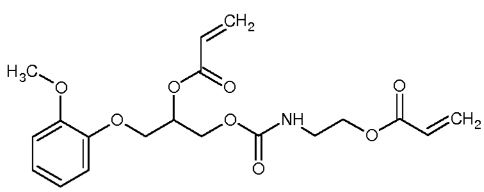

Beispiel 3.10

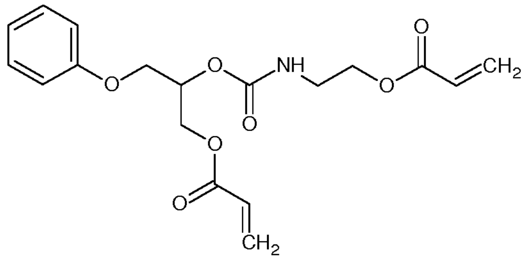

Beispiel 3.11

**Vergleichsbeispiel V1:** Phosphorothioyltris(oxybenzol-4,1-diylcarbamoyloxyethan-2,1-diyl)trisacrylat [1072455-04-9]

In einem 500 mL Rundkolben wurden 0.1 g 2,6-Di-tert.-butyl-4-methylphenol, 0.05 g Dibutylzinndilaurat sowie 213.07 g einer 27 %-igen Lösung von Tris(p-isocyanatophenyl)thiophosphat in Ethylacetat vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten. Das erhaltene Produkt hat einen $n^D_{20}$= 1,5430 (589nm).

**Vergleichsbeispiel V2:** Benzol-1,3-diylbis[oxy-3-(biphenyl-4-yloxy)propan-1,2-diyl]bisacrylat

Das Vergleichsbeispiel 2 wird in einer ersten Stufe aus einem Dithiol und ein Oxiran hergestellt (vgl. auch Beispiel 4a+b in der WO 2012/020061 A1)

Das Oxiran und der Katalysator wurden in einem Dreihalskolben vorgelegt, der mit KPG-Rührer und Rührmotor sowie Trockenrohr ausgerüstet war. Es wurde auf 60 bis 80°C temperiert und dann das Dithiol zugetropft. Anschließend wurde bei der angegebenen Temperatur weitergerührt, bis im $^1$H-NMR-Spektrum ein Umsatz der Oxirangruppe von >95% erkennbar war bzw. keine Oxirangruppen mehr nachweisbar waren.

| | |
|---|---|
| Stufe 1 | 3,3'-(Butan-2,3-diyldisulfanediyl)bis[1-(biphenyl-2-yloxy)propan-2 ol] |
| Edukte: | 14,3 g 2-[(Biphenyl-2-yloxy)methyl]oxiran (Denacol EX 142) |
| | 36 mg 1-Butyl-3-Methylimidazoliumbromid |
| | 3,7 g 2,3-Butandithiol |
| Bedingungen: | Reaktionstemperatur 60 °C beim Zutropfen, 80 °C, Reaktionszeit 48,5 h |
| | Es wurde eine klare, farblose, viskose Flüssigkeit erhalten. |
| $^1$H-NMR | ($CDCl_3$, 400 MHz): $\delta$ (1H)= 7,45 (d, 2H), 7,38 (t, 2H), 7,32 (m, 3H), 7,16 (t, 1H), 6,97 (d, 1H), 4,05 (d, 2H), 3,95 (m, 1H), 2,65 (dd, 1H), 2,58 (dd, 1H), 2,4-2,55 (m, 2H), 1,5-1,65 (m, 2H) |

**Stufe 2:** 6,13-Bis[(biphenyl-2-yloxy)methyl]-9,10-dimethyl-4,15,20-trioxo-5,14,19-trioxa-8,11-dithia-3,16-diazadocos-21-en-1-ylacrylat

Stufe 1, Dibutylzinndilaurat und 2,6-Di-tert.-butyl-4-methylphenol wurden in einem Dreihalskolben vorgelegt, der mit KPG-Rührer und Rührmotor, Gaseinlass sowie Trockenrohr ausgerüstet war. Anschließend wurde auf 60 °C temperiert, langsam Luft übergeleitet und das 2-Isocyanatoethylacrylat innerhalb ca. einer halben Stunde zugetropft. Es wurde weiter gerührt bis im IR-Spektrum keine NCO-Bande (2261cm$^{-1}$) mehr beobachtet werden konnte.

| | |
|---|---|
| Edukte: | 18,0 g Produkt aus Stufe 1 |
| | 8,5 g 2-Isocyanatoethylacrylat |
| | 13 mg Dibutylzinndilaurat |
| | 3 mg 2,6-Di-tert.-butyl-4-methylphenol |
| Bedingungen: | Zutropfen (exotherm!) in 35 Minuten bei 60 °C, |
| | dann Reaktionszeit von 16 h bei 60 °C |
| | Es wurde ein klares, fast farbloses, hochviskoses Produkt erhalten. |
| $n_D^{20}$ | 1,5840 (589nm) |

**Vergleichsbeispiele V3-V5:**

Die Vergleichsbeispiel 4-6 wurden analog Vergleichsbeispiel 3 aus der WO 2012/020061A1 hergestellt und ihre Viskosität gemessen. Die Ergebnisse sind in Tabelle 2 gezeigt.

Tabelle 2: Nicht erfinderische Vergleichbeispiele 4-6

| Vergleichsbeispiel | Nicht erfinderische Verbindung | Beispiel aus der WO 2012/020061 A1 | Viskosität |
|---|---|---|---|
| V3 | 6,16-Bis[(biphenyl-2-yloxy) methyl]-4,18,23-trioxo-5,11,17,22-tetraoxa-8,14-dithia-3,19-diazapentacos-24-en-1-ylacrylat | 6b | >1000000 mPas |
| V4 | 6,13-Bis[(biphenyl-2-yloxy) methyl]-4,15,20-trioxo-10-[({[2-(phenylsulfanyl)phenyl] carbamoyl}oxy) methyl]-5,14,19-trioxa-8,11-dithia-3,16-diazadocos-21-en-1-ylacrylat | 10b | >1000000 mPas |
| V5 | 6,19-Bis[(biphenyl-2-yloxy) methyl]-4,21,26-trioxo-5,11,14,20,25-pentaoxa-8,17-dithia-3,22-diazaoctacos-27-en-1-ylacrylat | 7b | >1000000 mPas |

**Polyol-Komponente:**

In einem 1 L Kolben wurden 0.18 g Addocat® SO, 374.8 g ε-Caprolacton und 374.8 g eines difunktionellen Polytetrahydrofuranpolyetherpolyols (Equivalentgewicht 650 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

**Urethanacrylat 1:** 2-({[3-(Methylsulfanyl)phenyl]carbamoyl}oxy)ethylprop-2-enoat

In einem 100 mL Rundkolben wurden 0,02 g 2,6-Di-tert.-butyl-4-methylphenol, 0,01 g Desmorapid Z, 11,7 g 3-(Methylthio)phenylisocyanat vorgelegt und auf 60 °C erwärmt. Anschließend wurden 8,2 g 2-Hydroxyethylacrylat zugetropft und die Mischung weiter auf 60 °C gehalten, bis der Isocyanatgehalt unter 0,1 % gesunken war. Danach wurde abgekühlt. Das Produkt wurde als farblose Flüssigkeit erhalten.

**Fluoriertes Urethan:** Bis(2,2,3,3,4,4,5,5,6,6,7,7-dodecafluoroheptyl)-(2,2,4-trimethyl-hexane-1,6-diyl)biscarbamat

In einem 6L-Rundkolben wurden 0,50 g Desmorapid Z und 1200 g Trimethylhexamethylendiisocyanat vorgelegt und auf 80 °C erwärmt. Anschließend wurden 3798 g 1H,1H,7H-Perfluorheptan-1-ol zugetropft und die Mischung weiter auf 80 °C gehalten, bis der Isocyanatgehalt unter 0,1 % gesunken war. Danach wurde abgekühlt. Das Produkt wurde als farbloses Öl erhalten.

**Herstellung der erfindungsgemäßen und nicht erfindungsgemäßen Medien (Coupons) (Beispiele 4.1 bis 4.11 und Vergleichbeispiele V4.1-V4.5)**

2,940 g der oben beschriebenen Polyol-Komponente wurden mit 2.000 g des jeweiligen Schreibmonomers (Beispiele 3.1 bis 3.11, Vergleichsbeispiele V4.1 - V4.5), 2,000 g des oben beschriebenen Urethanacrylats 1, 2,000 g des oben beschriebenen fluorierten Urethans, 0,15 g CGI 909, 15 mg Kristallviolett, 15 mg Irgacure 250, 15 mg Glasperlen der Größe 9,18 μm und 0,517 g g N-Ethylpyrolidon bei 60 °C gemischt, so dass eine klare (in manchen Fällen leicht trübe) Lösung erhalten wurde. Anschließend wurde auf 30 °C abgekühlt, 545 mg Desmodur® N 3900 zugegeben und erneut gemischt. Schließlich wurden 6 mg Fomrez UL 28 zugegeben und erneut kurz gemischt. Die erhaltene flüssige Masse wurde dann auf eine Glasplatte (Fa. Corning, NY 14831, USA, Micro slide plane: Dicke 0,96-1,06mm, 75mm x 50mm, Type: 2947-75x50) gegeben und dort mit einer zweiten Glasplatte abgedeckt. Dieser Probenkörper wurde 12 Stunden bei Raumtemperatur gelagert und dabei ausgehärtet. Anschließend wurden die Medien lichtdicht verpackt.

**Bestimmung der physikalischen Daten der erfindungsgemäßen und nicht erfindungsgemäßen Medien**

Die Messung der holographischen Eigenschaften DE und $\Delta n$ wurde gemäß dem oben im Abschnitt "Messmethoden" beschriebenen Verfahren durchgeführt.

Die Trübungsmessung wurde ebenfalls gemäß dem oben im Abschnitt "Messmethoden" beschriebenen Verfahren durchgeführt, wobei vor der Messung das jeweilige Medium zunächst bei Raumtemperatur und Umgebungslicht ca. 15-30 Minuten gebleicht wurde, bis die Farbe visuell nicht mehr zu erkennen war.

Die Ergebnisse der Messungen sind in der Tabelle 3 dargestellt:

Tabelle 3: Holografische und optische Performance der erfindungsgemäßen Beispiele der Photopolymerformulierungen 4.1-4.11 und der Vergleichsbeispiele V4.1 bis V4.5

| Erfindungsgemäße Beispiele | Schreibmonomer | Zeit zum Lösen | $\Delta n$ bei 16 $mJ/cm^2$ | d | Haze in % |
|---|---|---|---|---|---|
| | | [min] | [-] | [$\mu$m] | [%] |
| 4.1 | 3.1 | 0,50 | 0,0310 | 9,6 | 0,6 |
| 4.2 | 3.2 | 0,50 | 0,0275 | 10,8 | 0,7 |
| 4.3 | 3.3 | 0,33 | 0,0278 | 10,2 | 0,9 |
| 4.4 | 3.4 | 0,50 | 0,0370 | 7,9 | 0,4 |
| 4.5 | 3.5 | 1,00 | 0,0250 | 12,0 | 0,8 |
| 4.6 | 3.6 | 0,75 | 0,0250 | 10,7 | 2,0 |
| 4.7 | 3.7 | 2,00 | 0,0265 | 10,8 | 0,9 |
| 4.8 | 3.8 | 2,00 | 0,0358 | 9,4 | 1,1 |
| 4.9 | 3.9 | 0,50 | 0,0365 | 11,2 | 0,9 |
| 4.10 | 3.10 | 0,75 | 0,0310 | 11,8 | 0,6 |
| 4.11 | 3.11 | 0,75 | 0,0300 | 6,5 | 1,4 |
| Vergleichsbeispiele | | | | | |
| V4.1 | V1 | > 2800 | 0,0240 | 9,2 | 54,9 |
| V4.2 | V2 | 480 | 0,0315 | 10,0 | 1,0 |
| V4.3 | V3 | 70 | 0,0292 | 12,2 | 1,4 |
| V4.4 | V4 | > 480 | 0,0280 | 13,7 | 3,5 |
| V4.5 | V5 | 25 | 0,0290 | 10,0 | 0,9 |

Wie aus der Tabelle 3 hervorgeht, zeigten die holografischen Medien, bestehend aus erfindungsgemäßen Formulierungen enthaltend ein Schreibmonomer gemäß Formel (1) vergleichbare bzw. bessere holographische Performance für Transmissionshologramme von mehr als $\Delta n > 0{,}02$. Darüber hinaus sind die erfinderischen Beispiele zur Herstellung trübungsarmer holographischer Medien geeignet und zeigen bei Schichtdicken größer 6 $\mu$m einen Haze von weniger als 5%. Bei den erfindungsmäßen Beispiele 4.1-4.11 lösen sich die Schreibmonomere schnell und einfach ohne Zusatz von Lösemitteln innerhalb von weit weniger als 5 Minuten, während die Schreibmonomere der Vergleichsbeispiele V1-V5 deutlich länger benötigten, bevor sie vollständig gelöst sind. Folglich ist die Herstellung der Photopolymer-Formulierungen bei den Vergleichsbeispielen wesentlich zeitaufwendiger.

## Patentansprüche

1. Photopolymer-Formulierung umfassend

A) als Schreibmonomer mindestens einen aromatischer Glycolether der allgemeinen Formel (I)

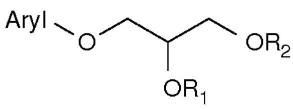

(I)

in der Aryl ein substituierter oder unsubstituierter aromatischer Rest ist, und $R_1$ und $R_2$ jeweils unabhängig voneinander ein Rest der Formel (II) oder (III)

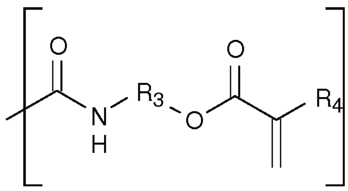

(II)

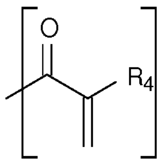

(III)

sind, in denen

$R_3$ ein organischer Rest mit bis zu 6 Kohlenstoffatomen ist, der Sauerstoff- und / oder Schwefelatome enthalten kann, und
$R_4$ ein Rest ausgewählt aus der Gruppe -H, $-CH_3$ ist;

B) Matrixpolymere;
C) einen Photoinititiator.

**2.** Photopolymer-Formulierung nach Anspruch 1, **dadurch gekennzeichnet, dass** der Rest Aryl 5 bis 21, bevorzugt 5 bis 18, weiter bevorzugt 6 bis 16 und besonders bevorzugt 6 bis 12 und ganz besonders bevorzugt 6 bis 10 Kohlenstoff- und / oder Heteroatome im aromatischen System umfasst.

**3.** Photopolymer-Formulierung nach einem der Ansprüche 1 oder 2, **dadurch gekennzeichnet, dass** der Rest Aryl mit 1 bis 5 und bevorzugt 1 bis 3 gleichen oder unterschiedlichen Substituenten ausgewählt aus der Gruppe n-Alkyl, verzweigtes Alkyl, Alkyloxy, Phenyl, Benzyl, Phenylalkyl, Naphtyl, Methylthiyl, Ethylthiyl, Alkylthiyl, Alkylthioalkyl, Phenoxy, Phenylthiyl, Naphtylthiyl, Fluor, Chlor, Brom und/oder Iod und bevorzugt mit 1 bis 3 gleichen oder unterschiedlichen Substituenten ausgewählt aus der Gruppe Methyl, Ethyl, Thiomethyl, Methoxy, Phenyl substituiert ist.

**4.** Photopolymer-Formulierung nach Anspruch 1, **dadurch gekennzeichnet, dass** der Rest Aryl ausgewählt aus der Gruppe Phenyl, Methylphenyl, Ethylphenyl, Thiomethylphenyl, Methoxyphenyl, Biphenyl und Naphtyl ist.

**5.** Photopolymer-Formulierung nach einem der Ansprüche 1 bis 4, **dadurch gekennzeichnet, dass** $R_3$ ein Rest ausgewählt aus der Gruppe $-CH_2-$, $-CH_2CH_2-$,$-CH(CH_3)CH_2-$, $-CH_2CH(CH_3)-$, $-CH_2CH_2CH_2-$, $-CH_2CH_2CH_2CH_2-$,$-CH_2CHOCH_2CH_2-$, $-CH_2CH_2OCH_2CH_2OCH_2CH_2-$ und bevorzugt ein Rest ausgewählt aus der Gruppe $-CH_2CH_2-$, $-CH(CH_3)CH_2-$, $-CH_2CH(CH_3)-$, $-CH_2CH_2\ CH_2CH_2-$, und ganz besonders bevorzugt $-CH_2CH_2-$ ist.

**6.** Photopolymer-Formulierung nach einem der Ansprüche 1 bis 5, **dadurch gekennzeichnet, dass** der Rest $R_1$ ein Rest der Formel (II) und der Rest $R_2$ ein Rest der Formel (III) ist, wobei der Rest $R_3$ insbesondere ein $-CH_2CH_2-$ Rest sein kann.

**7.** Photopolymer-Formulierung nach einem der Ansprüche 1 bis 5, **dadurch gekennzeichnet, dass** der Rest $R_1$ ein Rest der Formel (III) ist und der Rest $R_2$ ein Rest der Formel (II) ist, wobei der Rest $R_3$ insbesondere ein $-CH_2CH_2-$

Rest sein kann.

**8.** Photopolymer-Formulierung nach einem der Ansprüche 1 bis 7, **dadurch gekennzeichnet, dass** die Matrixpolymere B) vernetzt sind.

**9.** Photopolymer-Formulierung nach einem der Ansprüche 1 bis 8, **dadurch gekennzeichnet, dass** die Matrixpolymere Polyurethane und bevorzugt auf Polyester-C4-Polyetherpolyolen basierende Polyurethane sind.

**10.** Photopolymer-Formulierung nach einem der Ansprüche 1 bis 9, **dadurch gekennzeichnet, dass** sie zusätzlich monomere Fluorurethane umfasst.

**11.** Photopolymer-Formulierung nach Anspruch 10, **dadurch gekennzeichnet, dass** die Fluorurethane wenigstens eine Verbindung der Formel (IV)

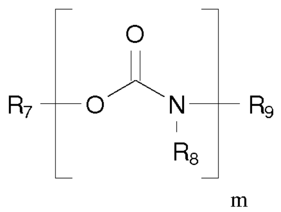

(IV)

umfassen oder daraus bestehen, in der $m \geq 1$ und $m \leq 8$ ist und $R_7$, $R_8$, $R_9$ unabhängig voneinander Wasserstoff, lineare, verzweigte, cyclische oder heterocyclische unsubstituierte oder gegebenenfalls auch mit Heteroatomen substituierte organische Reste sind, wobei bevorzugt mindestens einer der Reste $R_7$, $R_8$, $R_9$ mit wenigstens einem Fluoratom substituiert ist und besonders bevorzugt $R_7$ ein organischer Rest mit mindestens einem Fluoratom ist.

**12.** Holographisches Medium, enthaltend eine Photopolymer-Formulierung nach einem der Ansprüche 1 bis 11 oder erhältlich unter Verwendung einer Photopolymer-Formulierung nach einem der Ansprüche 1 bis 11.

**13.** Holographisches Medium nach Anspruch 12, in dass wenigstens ein Hologramm einbelichtet ist.

**14.** Holographisches Medium nach Anspruch 13, **dadurch gekennzeichnet, dass** das Hologramm ein Reflektions-, Transmissions-, In-Line-, Off-Axis-, Full-Aperture Transfer-, Weißlicht-Transmissions-, Denisyuk-, Off-Axis Reflektions- oder Edge-Lit Hologramm sowie ein holographisches Stereogramm, bevorzugt Reflektions-, Transmissions- oder Edge-Lit Hologramm ist.

**15.** Optische Anzeige umfassend ein holographisches Medium nach Anspruch 13 oder 14.

**16.** Verwendung eines holographischen Mediums nach Anspruch 13 oder 14 zur Herstellung von Chipkarten, Ausweisdokumenten, 3D-Bildern, Produktschutzetiketten, Labeln, Banknoten oder holografisch optischen Elementen insbesondere für optische Anzeigen.

**17.** Aromatischer Glycolether der Formel (I)

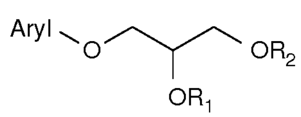

(I)

in der Aryl ein substituierter oder unsubstituierter aromatischer Rest, ausgeschlossen unsubstituiertes Phenyl, ist und $R_1$ und $R_2$ jeweils unabhängig voneinander ein Rest der Formel (II) oder (III)

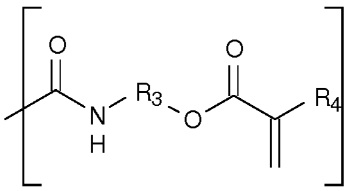

(II)

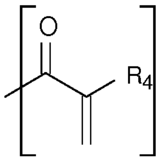

(III)

sind, in denen

$R_3$ ein organischer Rest mit bis zu 6 Kohlenstoffatomen ist, der Sauerstoff- und / oder Schwefelatome enthalten kann,
$R_4$ ein Rest ausgewählt aus der Gruppe -H, $-CH_3$ ist,

wobei Aryl ein Rest ausgewählt aus der Gruppe Methylphenyl, Ethylphenyl, Thiomethylphenyl, Methoxyphenyl, Biphenyl und Naphtyl ist

**18.** Aromatischer Glycolether gemäß Anspruch 17, **dadurch gekennzeichnet, dass** jeweils einer der Reste $R_1$ und $R_2$ ein Rest der Formel (II) und jeweils einer der Reste $R_1$ und $R_2$ ein Rest der Formel (III) ist, wobei $R_3$ insbesondere ein $-CH_2CH_2-$ Rest sein kann, bevorzugter wobei Rest $R_1$ ein Rest der Formel (III) und der Rest $R_2$ ein Rest der Formel (II) ist.

## Claims

**1.** Photopolymer formulation comprising

A) as writing monomer at least one aromatic glycol ether of the general formula (I)

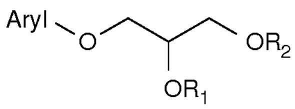

(I)

in which aryl is a substituted or unsubstituted aromatic radical, and
$R_1$ and $R_2$ are each independently a radical of the formula (II) or (III)

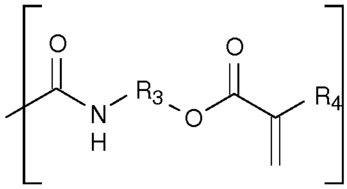

(II)

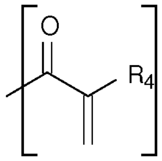
(III)

in which

$R_3$ is an organic radical which has up to 6 carbon atoms and may contain oxygen and/or sulphur atoms, and $R_4$ is a radical selected from the group of -H, $-CH_3$;

B) matrix polymers;
C) a photoinitiator.

2. Photopolymer formulation according to Claim 1, **characterized in that** the aryl radical comprises 5 to 21, preferably 5 to 18, further preferably 6 to 16 and more preferably 6 to 12 and most preferably 6 to 10 carbon atoms and/or heteroatoms in the aromatic system.

3. Photopolymer formulation according to either of Claims 1 and 2, **characterized in that** the aryl radical is substituted by 1 to 5 and preferably 1 to 3 identical or different substituents selected from the group of n-alkyl, branched alkyl, alkyloxy, phenyl, benzyl, phenylalkyl, naphthyl, methylthiyl, ethylthiyl, alkylthiyl, alkylthioalkyl, phenoxy, phenylthiyl, napthylthiyl, fluorine, chlorine, bromine and/or iodine, and preferably by 1 to 3 identical or different substituents selected from the group of methyl, ethyl, thiomethyl, methoxy, phenyl.

4. Photopolymer formulation according to Claim 1, **characterized in that** the aryl radical is selected from the group of phenyl, methylphenyl, ethylphenyl, thiomethylphenyl, methoxyphenyl, biphenyl and naphthyl.

5. Photopolymer formulate on according to any of Claims 1 to 4, **characterized in that** $R_3$ is a radical selected from the group of $-CH_2-$, $-CH_2CH_2-$,$-CH(CH_3)CH_2-$, $-CH_2CH(CH_3)-$, $-CH_2CH_2CH_2-$, $-CH_2CH_2CH_2CH_2-$, $-CH_2CHOCH_2CH_2-$, $-CH_2CH_2OCH_2CH_2OCH_2CH_2-$ and preferably a radical selected from the group of $-CH_2CH_2-$, $-CH(CH_3)CH_2-$, $-CH_2CH(CH_3)-$, $-CH_2CH_2CH_2CH_2-$, and most preferably $-CH_2CH_2-$.

6. Photopolymer formulation according to any of Claims 1 to 5, **characterized in that** the $R_1$ radical is a radical of the formula (II) and the $R_2$ radical is a radical of the formula (III), where the $R_3$ radical may especially be a $-CH_2CH_2-$ radical.

7. Photopolymer formulation according to any of Claims 1 to 5, **characterized in that** the $R_1$ radical is a radical of the formula (III) and the $R_2$ radical is a radical of the formula (II), where the $R_3$ radical may especially be a $-CH_2CH_2-$ radical.

8. Photopolymer formulation according to any of Claims 1 to 7, **characterized in that** the matrix polymers B) have been crosslinked.

9. Photopolymer formulation according to any of Claims 1 to 8, **characterized in that** the matrix polymers are polyurethanes and preferably polyurethanes based on polyester C4 polyether polyols.

10. Photopolymer formulation according to any of Claims 1 to 9, **characterized in that** it additionally comprises monomeric fluoroethanes.

11. Photopolymer formulation according to Claim 10, **characterized in that** the fluorourethanes comprise or consist of at least one compound of the formula (IV)

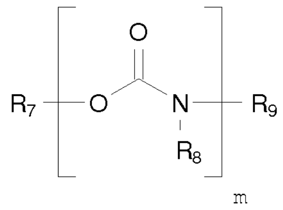

(IV)

in which m ≥ 1 and m ≤ 8 and $R_7$, $R_8$, $R_9$ are each independently hydrogen or linear, branched, cyclic or heterocyclic organic radicals which are unsubstituted or else optionally substituted by heteroatoms, where preferably at least one of the $R_7$, $R_8$, $R_9$ radicals is substituted by at least one fluorine atom and, more preferably, $R_7$ is an organic radical having at least one fluorine atom.

**12.** Holographic medium comprising a photopolymer formulation according to any of Claims 1 to 11 or obtainable using a photopolymer formulation according to any of Claims 1 to 11.

**13.** Holographic medium according to Claim 12 into which at least one hologram has been exposed.

**14.** Holographic medium according to Claim 13, **characterized in that** the hologram is a reflection, transmission, in-line, off-axis, full-aperture transfer, white light transmission, Denisyuk, off-axis reflection or edge-lit hologram, or else a holographic stereogram, preferably a reflection, transmission or edge-lit hologram.

**15.** Visual display comprising a holographic medium according to Claim 13 or 14.

**16.** Use of a holographic medium according to Claim 13 or 14 for production of chip cards, identification documents, 3D images, product protection labels, labels, banknotes or holographic optical elements, especially for visual displays.

**17.** Aromatic glycol ethers of the formula (I)

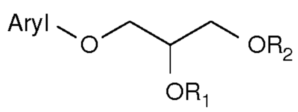

(I)

in which aryl is a substituted or unsubstituted aromatic radical, excluding unsubstituted phenyl, and
$R_1$ and $R_2$ are each independently a radical of the formula (II) or (III)

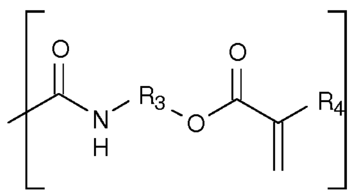

(II)

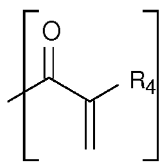

(III)

in which

$R_3$ is an organic radical which has up to 6 carbon atoms and may contain oxygen and/or sulphur atoms,
$R_4$ is a radical selected from the group of -H, - $CH_3$,

where aryl is a radical selected from the group of methylphenyl, ethylphenyl, thiomethylphenyl, methoxyphenyl, biphenyl and naphthyl.

**18.** Aromatic glycol ether according to Claim 17, **characterized in that** in each case one of the $R_1$ and $R_2$ radicals is a radical of the formula (II) and in each case one of the $R_1$ and $R_2$ radicals is a radical of the formula (III), where $R_3$ may especially be a $-CH_2CH_2-$ radical, more preferably where $R_1$ radical is a radical of the formula (III) and the $R_2$ radical is a radical of the formula (II).

**Revendications**

**1.** Formulation de photopolymère, comprenant :

A) en tant que monomère d'écriture, au moins un éther de glycol aromatique de formule générale (I)

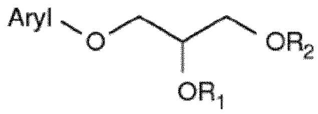

(I)

dans laquelle aryle est un radical aromatique substitué ou non substitué, et
$R_1$ et $R_2$ représentent chacun indépendamment l'un de l'autre un radical de formule (II) ou (III)

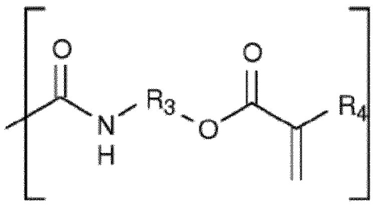

(II)

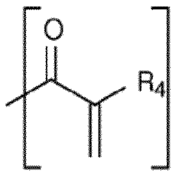

(III)

dans lesquelles

$R_3$ est un radical organique contenant jusqu'à 6 atomes de carbone, qui peut contenir des atomes d'oxygène et/ou de soufre, et
$R_4$ est un radical choisi dans le groupe constitué par -H, $-CH_3$ ;

B) des polymères de matrice ;
C) un photoinitiateur.

**2.** Formulation de photopolymère selon la revendication 1, **caractérisée en ce que** le radical aryle comprend 5 à 21, de préférence 5 à 18, de manière davantage préférée 6 à 16 et de manière particulièrement préférée 6 à 12 et de manière tout particulièrement préférée 6 à 10 atomes de carbone et/ou hétéroatomes dans le système aromatique.

**3.** Formulation de photopolymère selon l'une quelconque des revendications 1 ou 2, **caractérisée en ce que** le radical

aryle est substitué avec 1 à 5, et de préférence 1 à 3 substituants identiques ou différents choisis dans le groupe constitué par n-alkyle, alkyle ramifié, alkyloxy, phényle, benzyle, phénylalkyle, naphtyle, méthylthiyle, éthylthiyle, alkylthiyle, alkylthioalkyle, phénoxy, phénylthiyle, naphtylthiyle, fluor, chlore, brome et/ou iode, et de préférence avec 1 à 3 substituants identiques ou différents choisis dans le groupe constitué par méthyle, éthyle, thiométhyle, méthoxy, phényle.

**4.** Formulation de photopolymère selon la revendication 1, **caractérisée en ce que** le radical aryle est choisi dans le groupe constitué par phényle, méthylphényle, éthylphényle, thiométhylphényle, méthoxyphényle, biphényle et naphtyle.

**5.** Formulation de photopolymère selon l'une quelconque des revendications 1 à 4, **caractérisée en ce que** $R_3$ est un radical choisi dans le groupe constitué par $-CH_2-$, $-CH_2CH_2-$, $-CH(CH_3)CH_2-$, $-CH_2CH(CH_3)-$,$-CH_2CH_2CH_2-$, $-CH_2CH_2CH_2CH_2-$, $-CH_2CHOCH_2CH_2-$,$-CH_2CH_2OCH_2CH_2OCH_2CH_2-$, et de préférence un radical choisi dans le groupe constitué par $-CH_2CH_2-$, $-CH(CH_3)CH_2-$,$-CH_2CH(CH_3)-$, $-CH_2CH_2CH_2CH_2-$, et de manière tout particulièrement préférée $-CH_2CH_2-$.

**6.** Formulation de photopolymère selon l'une quelconque des revendications 1 à 5, **caractérisée en ce que** le radical $R_1$ est un radical de formule (II) et le radical $R_2$ est un radical de formule (III), le radical $R_3$ pouvant notamment être un radical $-CH_2CH_2-$.

**7.** Formulation de photopolymère selon l'une quelconque des revendications 1 à 5, **caractérisée en ce que** le radical $R_1$ est un radical de formule (III) et le radical $R_2$ est un radical de formule (II), le radical $R_3$ pouvant notamment être un radical $-CH_2CH_2-$.

**8.** Formulation de photopolymère selon l'une quelconque des revendications 1 à 7, **caractérisée en ce que** les polymères de matrice B) sont réticulés.

**9.** Formulation de photopolymère selon l'une quelconque des revendications 1 à 8, **caractérisée en ce que** les polymères de matrice sont des polyuréthanes et de préférence des polyuréthanes à base de polyester-polyéther-polyols en C4.

**10.** Formulation de photopolymère selon l'une quelconque des revendications 1 à 9, **caractérisée en ce qu'**elle comprend en outre des fluorouréthanes monomères.

**11.** Formulation de photopolymère selon la revendication 10, **caractérisée en ce que** les fluorouréthanes comprennent au moins un composé de formule (IV)

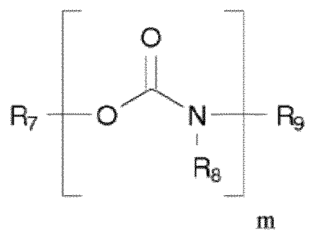

(IV)

ou en sont constitués, dans laquelle $m \geq 1$ et $m \leq 8$, et $R_7$, $R_8$, $R_9$ sont indépendamment les uns des autres l'hydrogène, des radicaux organiques linéaires, ramifiés, cycliques ou hétérocycliques non substitués ou éventuellement également substitués avec des hétéroatomes, au moins un des radicaux $R_7$, $R_8$, $R_9$ étant de préférence substitué avec au moins un atome de fluor, et $R_7$ étant de manière particulièrement préférée un radical organique contenant au moins un atome de fluor.

**12.** Support holographique, contenant une formulation de photopolymère selon l'une quelconque des revendications 1 à 11 ou pouvant être obtenu en utilisant une formulation de photopolymère selon l'une quelconque des revendications 1 à 11.

**13.** Support holographique selon la revendication 12, dans lequel au moins un hologramme est exposé.

**14.** Support holographique selon la revendication 13, **caractérisé en ce que** l'hologramme est un hologramme en réflexion, un hologramme en transmission, un hologramme en ligne, un hologramme désaxé, un hologramme de transfert à ouverture totale, un hologramme en transmission en lumière blanche, un hologramme de Denisyuk, un hologramme désaxé en réflexion ou un hologramme à bords éclairés, ainsi qu'un stéréogramme holographique, de préférence un hologramme en réflexion, un hologramme en transmission ou un hologramme à bords éclairés.

**15.** Affichage optique comprenant un support holographique selon la revendication 13 ou 14.

**16.** Utilisation d'un support holographique selon la revendication 13 ou 14 pour la fabrication de cartes à puce, de documents d'identité, d'images 3D, d'étiquettes de protection de produits, d'étiquettes, de billets de banque ou d'éléments optiques holographiques, notamment pour des affichages optiques.

**17.** Éther de glycol aromatique de formule (I)

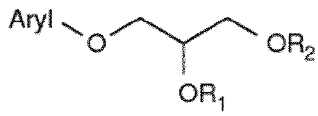

(I)

dans laquelle aryle est un radical aromatique substitué ou non substitué, à l'exclusion d'un phényle non substitué, et $R_1$ et $R_2$ représentent chacun indépendamment l'un de l'autre un radical de formule (II) ou (III)

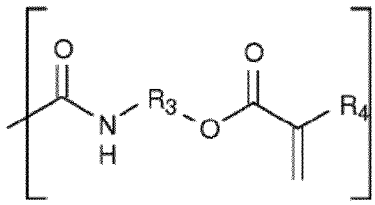

(II)

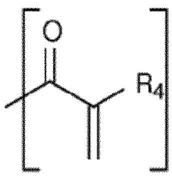

(III)

dans lesquelles

$R_3$ est un radical organique contenant jusqu'à 6 atomes de carbone, qui peut contenir des atomes d'oxygène et/ou de soufre,
$R_4$ est un radical choisi dans le groupe constitué par -H, $-CH_3$,

aryle étant un radical choisi dans le groupe constitué par méthylphényle, éthylphényle, thiométhylphényle, méthoxyphényle, biphényle et naphtyle.

**18.** Éther de glycol aromatique selon la revendication 17, **caractérisé en ce qu'**un des radicaux $R_1$ et $R_2$ est un radical de formule (II) et un des radicaux $R_1$ et $R_2$ est un radical de formule (III), $R_3$ pouvant notamment être un radical $-CH_2CH_2-$, le radical $R_1$ étant de manière davantage préférée un radical de formule (III) et le radical $R_2$ étant un radical de formule (II).

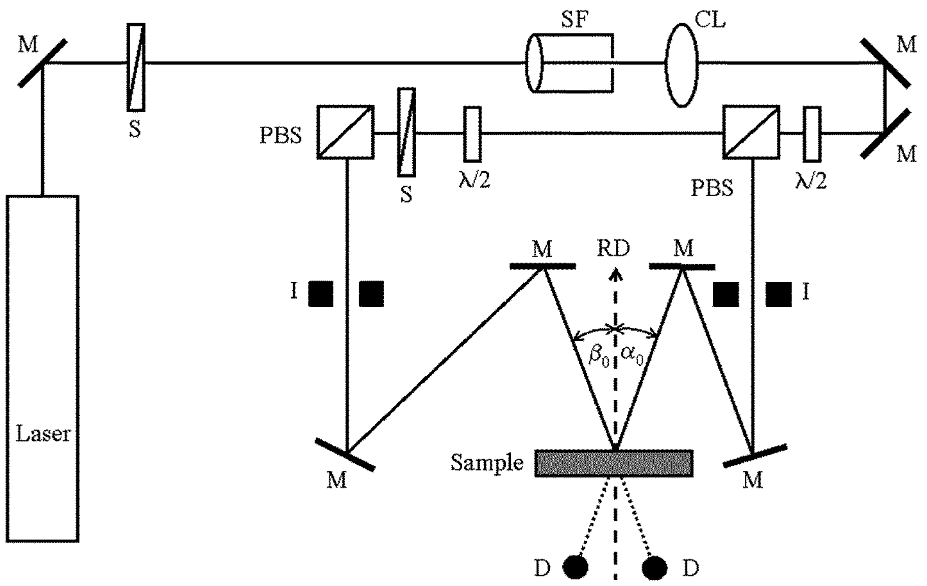


Figur 1

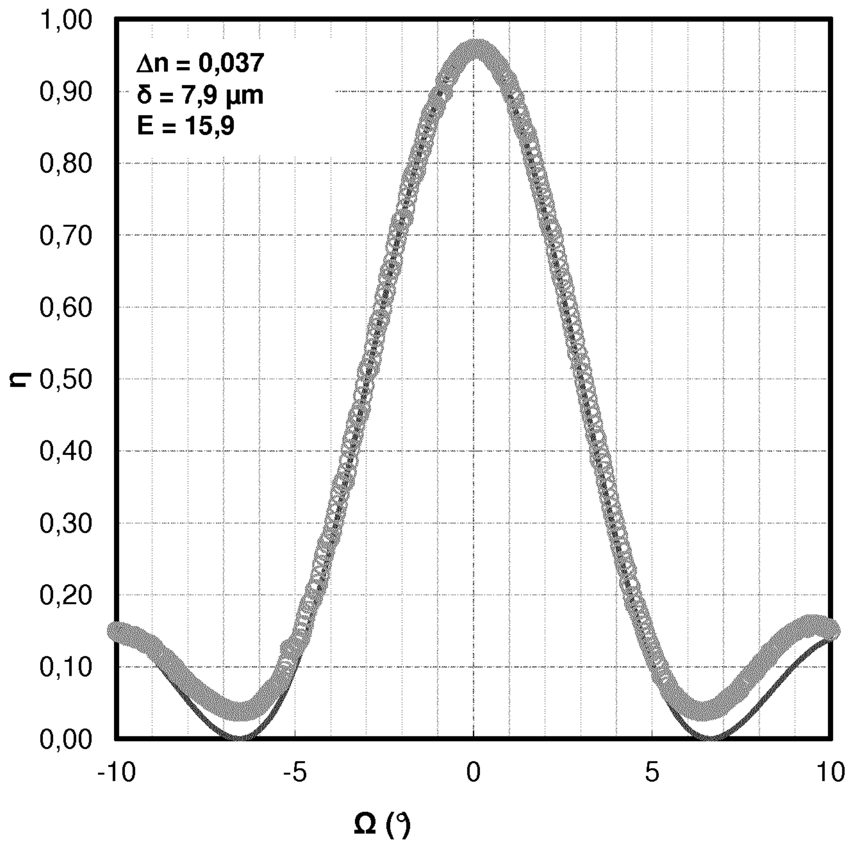


Figur 2

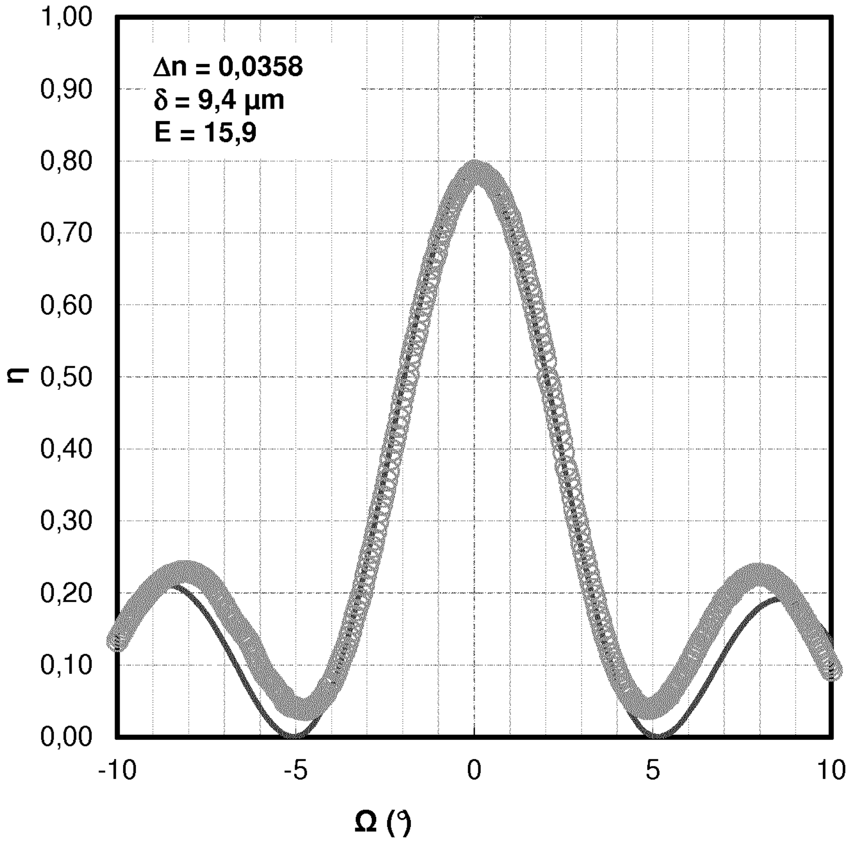
Δn = 0,0358
δ = 9,4 µm
E = 15,9
1,00
0,90
0,80
0,70
0,60
0,50
0,40
0,30
0,20
0,10
0,00
η
-10
-5
0
5
10
Ω (°)

Figur 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008125229 A
- WO 2012020061 A
- WO 2008125199 A
- EP 2401998 A1
- WO 2011095442 A2
- EP 1674940 A1
- US 4994347 A
- EP 0223587 A
- WO 2012062655 A
- US 20100036013 A1
- WO 2012020061 A1


### In der Beschreibung aufgeführte Nicht-Patentliteratur


- **P. HARIHARAN.** Optical Holography. Cambridge University Press, 1996
- **A BUKOWSKA et al.** *Journal of Molecular Catalysis A: Chemical,* 2005, vol. 225, 7-10
- **H. BERNETH.** Ullmann's Encyclopedia of Industrial Chemistry. Wiley-VCH Verlag, 2008
- **H. BERNETH.** Ullmann's Encyclopedia of Industrial Chemistry, Methine Dyes and Pigments. Wiley-VCH Verlag, 2008
- Triarylmethane and Diarylmethane Dyes. **T. GESSNER ; U. MAYER.** Ullmann's Encyclopedia of Industrial Chemistry. Wiley-VCH Verlag, 2000
- *J. Comput. Aid. Mol. Des.,* 2005, vol. 19, 453
- **CUNNINGHAM et al.** *RadTech'98 North America UV/EB Conference Proceedings,* 19. April 1998
- Chemistry & Technology of UV & EB Formulations For Coatings. Inks & Paints. SITA Technology, 1991, vol. 3, 61-328
- **H. KOGELNIK.** *The Bell System Technical Journal,* November 1969, vol. 48 (9), 2909-2947